# EUROPEAN PATENT APPLICATION

(11) **EP 3 812 036 A1**
(43) Date of publication of application: **28.04.2021**
(21) Application number: 19821654.1
(22) Date of filing: 07.06.2019
(51) Int. Cl.: B01J 13/00, A61K 8/04, A61K 8/42, A61K 9/107, A61K 47/18, A61K 47/32, B01F 17/16, B01F 17/52

(54) **NOVEL COMPOSITE AND EMULSION COMPOSITION**

(30) Priority: 20.06.2018 JP 2018116956
(71) Applicant: Kokyu Alcohol Kogyo Co., Ltd., Narita-shi, Chiba 287-0225 (JP)
(72) Inventor: OMURA Takayuki, Narita-shi Chiba 287-0225 (JP); HANADA, Naomi, Narita-shi, Chiba 287-0225 (JP)
(74) Representative: Cabinet Nuss
(86) International application number: PCT/JP2019/022754
(87) International publication number: WO 2019/244674

(57) **Abstract**

The purpose of the present invention is to provide a novel agent having emulsifying capability. A composite which is formed by combining an amide alcohol ester and a carboxyl group-containing polymer exhibits emulsifying power.

## Description

### [Technical Field]

The present invention relates to a novel complex having emulsifying properties and an emulsion composition.

### [Background Art]

Conventionally, as an emulsion composition for cosmetic materials, creams, milky lotions, beauty essences and the like, in which various oily and aqueous raw materials are emulsified, have been widely used. In the production of such an emulsion composition, it was necessary not only to process raw materials under various strictly set conditions but also to use a surfactant to guarantee the stability of emulsion compositions over time. However, in recent years, even higher safety is expected for cosmetic materials, and from this viewpoint, presence of a surfactant may be a problem.

For such problem, for example, a water-in-oil emulsion composition has been proposed, which comprises one or more oil components selected from the group consisting of fatty acids and higher alcohols, one or more polymers soluble in the oils, and one or more compounds selected from the group consisting of inorganic salts, organic acid salts, amino acids and salts thereof, and which is characterized in that it does not contain a surfactant (Patent Document 1). However, polyvinylpyrrolidone (PVP) used as one or more polymers soluble in the oil causes stickiness and has a problem in usability.

There still is a demand for new emulsion compositions that do not use a surfactant and for materials for preparing them.

### [Citation List]

### [Patent Document]

[Patent Document 1] JP A No. 2003-252723

### [Disclosure of Invention]

### [Problems to Be Solved by the Invention]

In view of the above-mentioned problems in the prior art, an object of the present invention is to provide an agent that exerts emulsifying power.

Furthermore, it is also an object of the present invention to provide an emulsion utilizing such emulsifying power. Another object of the present invention is to provide an agent capable of obtaining an emulsion having excellent stability as well as a stable emulsion. Furthermore, another object of the present invention is to provide an agent capable of obtaining an emulsion having excellent feeling of use for skin and hair, and an emulsion having excellent feeling of use.

### [Means for Solving the Problems]

While carrying out extensive research to solve the above-mentioned problems, the present inventors have discovered that emulsifying power is brought about by using an amide alcohol ester and a polymer containing a carboxyl group; and as a result of further research, the inventors have completed the present invention.

That is, the present invention relates to the following [1] to [13].
[1] A method for producing an emulsion composition, wherein an aqueous phase containing a carboxyl group-containing polymer, and an oil phase containing an amide alcohol ester represented by formula (I): 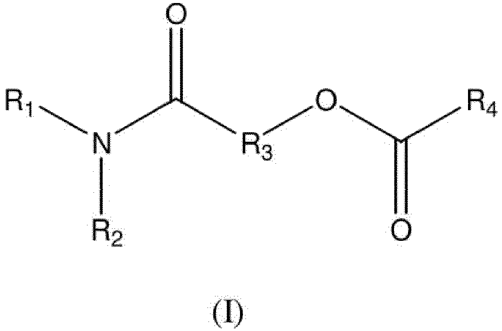 wherein
   R₁ is a C6-C22 hydrocarbon group that may be substituted,
   R₂ is H, or a C6-C22 hydrocarbon group that may be substituted,
   R₃ is a linear or branched C2-C21 hydrocarbon group that may be substituted,
   R₄ is a C1-C42 hydrocarbon group that may be substituted; or a group represented by the following formula (II): 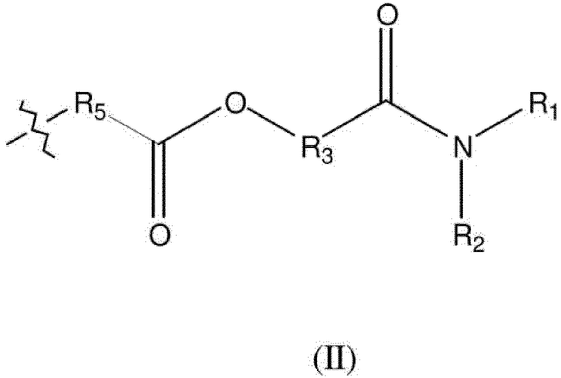 wherein in formula (II),
      R₁ to R₃ are the same as the above definitions,
      R₅ is a C2-C42 hydrocarbon group that may be substituted; are mixed.
[2] The method according to [1], which comprises neutralizing by adding a neutralizing agent.
[3] The method according to [2], wherein the aqueous phase containing the carboxyl group-containing polymer is neutralized by adding a neutralizing agent, and then the aqueous phase and the oil phase are mixed.
[4] The method according to [2], wherein, after mixing the aqueous phase and the oil phase, the mixture is neutralized by adding a neutralizing agent.
[5] The method according to any one of [1] to [4], wherein the carboxyl group-containing polymer has a molecular weight of 500,000 to 3,000,000 and a carboxyl group content of 50 to 70%.
[6] The method according to any one of [1] to [5], wherein the carboxyl group-containing polymer is a carboxin polymer and/or an alkyl-modified carboxyvinyl polymer.
[7] The method according to [6], wherein the carboxyl group-containing polymer is a carboxyvinyl polymer represented by formula (IV): 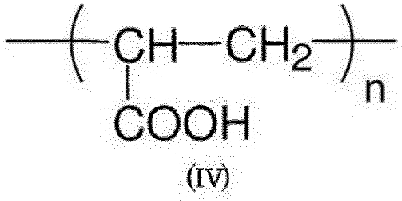 wherein n is an integer,
   and/or an alkyl-modified carboxyvinyl polymer represented by formula (V): 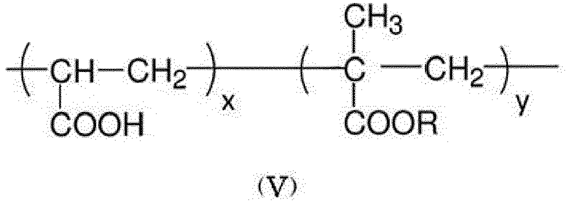 wherein x and y are each independently an integer, R is a C10-C30 alkyl group.
[8] The method according to any one of [1] to [7], wherein the amide alcohol ester is an amide alcohol ester of formula (I), wherein
   R₁ is a C10-C22 hydrocarbon group,
   R₂ is H,
   R₃ is a C3-C12 hydrocarbon group,
   R₄ is a C1-C22 hydrocarbon group.
[9] The method according to [8], wherein the amide alcohol is one or more selected from: 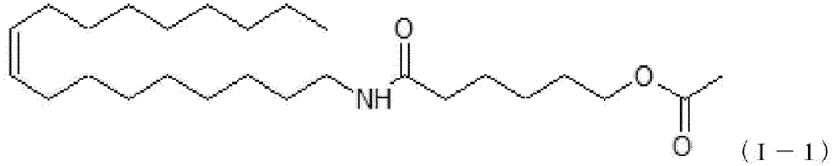 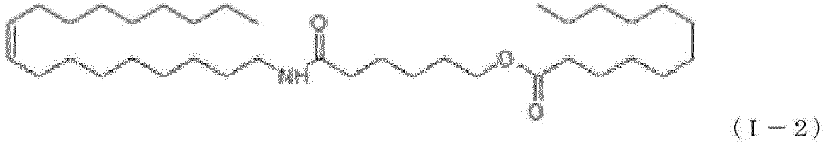 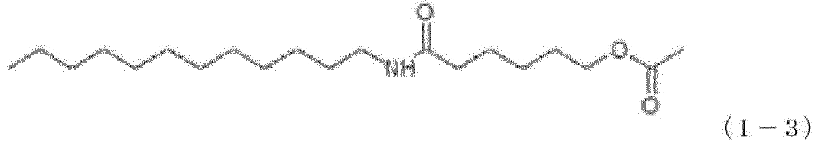 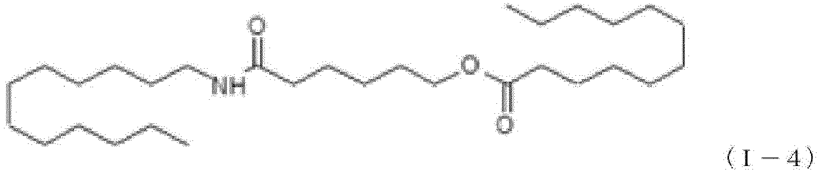 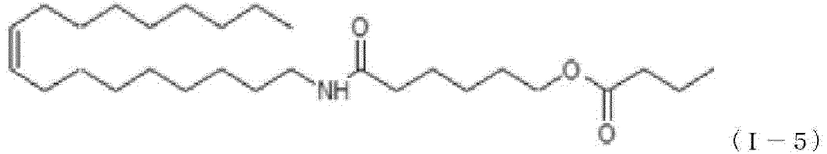
[10] An emulsion composition obtained by the method according to any one of [1] to [9].
[11] An agent containing an amide alcohol ester represented by formula (I): 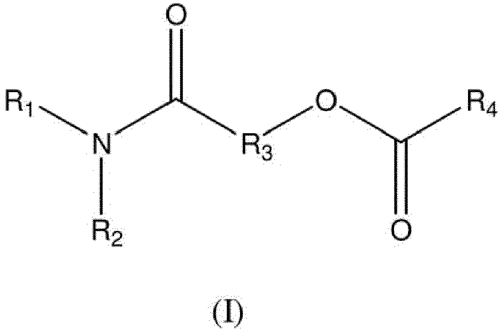 wherein
   R₁ is a C6-C22 hydrocarbon group that may be substituted,
   R₂ is H, or a C6-C22 hydrocarbon group that may be substituted,
   R₃ is a linear or branched C2-C21 hydrocarbon group that may be substituted,
   R₄ is a C1-C42 hydrocarbon group that may be substituted; or a group represented by the following formula (II): 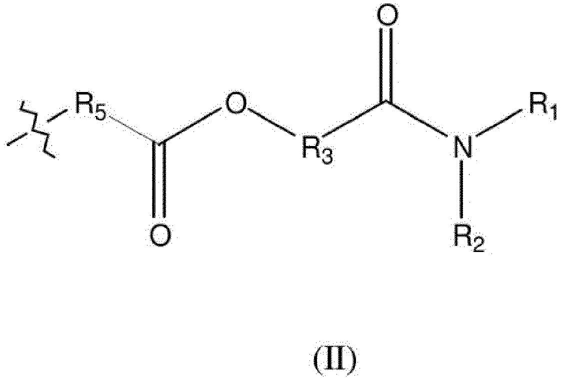 wherein in formula (II),
      R₁ to R₃ are the same as the above definitions,
      R₅ is a C2-C42 hydrocarbon group that may be substituted; and said agent forms a complex with a carboxyl group-containing polymer and is used for emulsification.
[12] An agent containing a carboxyl group-containing polymer, which forms a complex with an amide alcohol ester represented by formula (I): 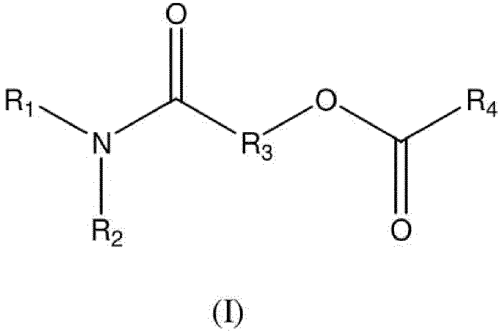 wherein
   R₁ is a C6-C22 hydrocarbon group that may be substituted,
   R₂ is H, or a C6-C22 hydrocarbon group that may be substituted,
   R₃ is a linear or branched C2-C21 hydrocarbon group that may be substituted,
   R₄ is a C1-C42 hydrocarbon group that may be substituted; or a group represented by the following formula (II): 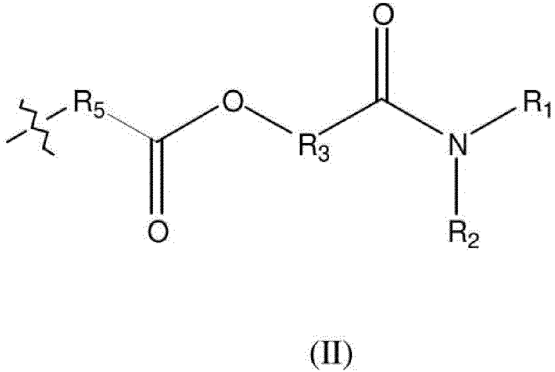 wherein in formula (II),
      R₁ to R₃ are the same as the above definitions,
      R₅ is a C2-C42 hydrocarbon group that may be substituted; and which is used for emulsification.
[13] A complex wherein a carboxyl group-containing polymer is bound with an amide alcohol ester represented by formula (I) : 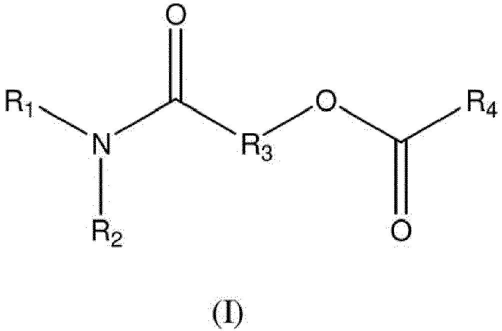 wherein
   R₁ is a C6-C22 hydrocarbon group that may be substituted,
   R₂ is H, or a C6-C22 hydrocarbon group that may be substituted,
   R₃ is a linear or branched C2-C21 hydrocarbon group that may be substituted,
   R₄ is a C1-C42 hydrocarbon group that may be substituted; or a group represented by the following formula (II): 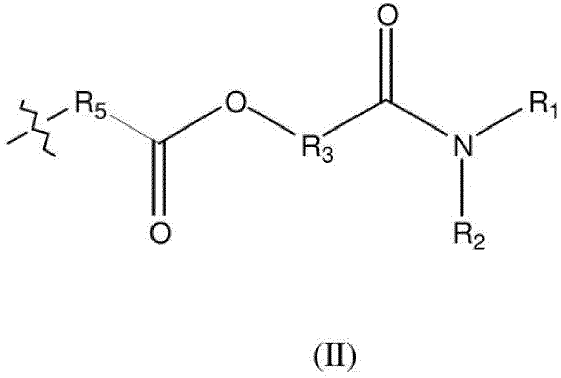 wherein in formula (II),
      R₁ to R₃ are the same as the above definitions,
      R₅ is a C2-C42 hydrocarbon group that may be substituted.

### [Advantageous Effects of Invention]

According to the present invention, it is possible to provide an agent and a complex that exert emulsifying power. In addition, according to the present invention, it is also possible to provide an emulsion utilizing such emulsifying power.

Furthermore, according to the present invention, an agent capable of obtaining an emulsion having excellent stability as well as a stable emulsion can be provided, and also an agent capable of obtaining an emulsion having excellent usability on the skin and hair, as well as an emulsion having excellent usability can be provided.

### [Brief Description of Drawings]

[Fig. 1] Figure 1 is a diagram showing an emulsified state of Examples 1A and 1B (O/W emulsion: post-addition, pre-addition).
[Fig. 2] Figure 2 is a diagram showing FT-IR measurement results of each of an amide alcohol ester, the formulation (Example 1A), and a carboxyl group-containing polymer.
[Fig. 3] Figure 3 is a diagram showing superimposed IR spectra of the formulation and an amide alcohol ester alone.
[Fig. 4] Figure 4 is a diagram showing superimposed IR spectra of the formulation and a carboxyl group-containing polymer alone.
[Fig. 5] Figure 5 is a diagram showing an emulsified state of Examples 2A and 2B (O/W emulsion: with and without addition of polyhydric alcohol).
[Fig. 6] Figure 6 is a diagram showing an emulsified state of Example 3 (O/W emulsion).
[Fig. 7] Figure 7 is a diagram showing an emulsified state of Example 4 (W/O emulsion) before and after addition of a neutralizing agent.
[Fig. 8] Figure 8 is a diagram showing FT-IR measurement results of each of an amide alcohol ester, the formulation (Example 4), and a carboxyl group-containing polymer.
[Fig. 9] Figure 9 is a diagram showing superimposed IR spectra of the formulation and an amide alcohol ester alone.
[Fig. 10] Figure 10 is a diagram showing superimposed IR spectra of the formulation and a carboxyl group-containing polymer alone.
[Fig. 11] Figure 11 is a diagram showing an emulsified state of Example 5A (W/O emulsion) before and after addition of a neutralizing agent.
[Fig. 12] Figure 12 is a diagram showing an emulsified state of Example 5B (W/O emulsion: pre-addition) immediately after emulsification and after cooling.
[Fig. 13] Figure 13 is a diagram showing an emulsified state of Example 5C (W/O emulsion) before and after addition of a neutralizing agent.
[Fig. 14] Figure 14 is a diagram showing an emulsified state of Example 5D (W/O emulsion: pre-addition) immediately after emulsification and after cooling.
[Fig. 15] Figure 15 is a diagram showing an emulsified state of Example 5E (W/O emulsion) before and after addition of a neutralizing agent.
[Fig. 16] Figure 16 is a diagram showing an emulsified state of Example 5F (W/O emulsion: pre-addition) immediately after emulsification and after cooling.

### [Embodiments for Carrying out the Invention]

### Component (A): Amide alcohol ester

The amide alcohol ester used in the present invention is a compound represented by the following formula (I): 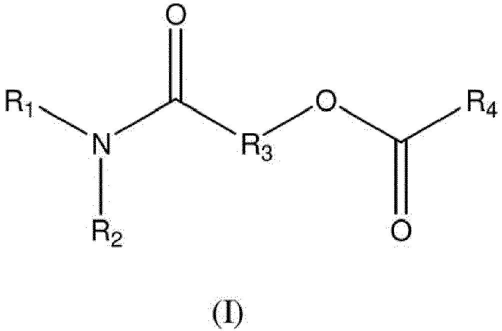 wherein
R₁ is a C6-C22 hydrocarbon group that may be substituted,
R₂ is H, or a C6-C22 hydrocarbon group that may be substituted,
R₃ is a linear or branched C2-C21 hydrocarbon group that may be substituted,
R₄ is a C1-C42 hydrocarbon group that may be substituted; or a group represented by the following formula (II): 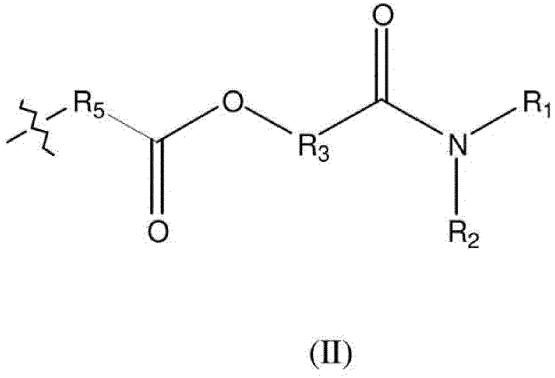 wherein in formula (II),
   R₁ to R₃ are the same as the above definitions,
   R₅ is a C2-C42 hydrocarbon group that may be substituted.

As used herein, the term "hydrocarbon group" may be saturated or unsaturated, linear or branched or cyclic, or a combination of linear or branched with cyclic, unless otherwise specified, and includes a hydrocarbon group consisting of a linear or branched hydrocarbon moiety such as benzyl group, phenylethyl group and a cyclic hydrocarbon moiety.

That is, the C6-C22 hydrocarbon group in R₁ and R₂ includes a saturated or unsaturated, linear, branched or cyclic C6-C22 hydrocarbon group, or a C6-C22 hydrocarbon group consisting of a saturated or unsaturated, linear or branched hydrocarbon moiety and a cyclic hydrocarbon moiety, and examples thereof include cyclic groups such as cyclohexyl, decahydronaphthyl, tetrahydrodicyclopentadiene, sterol, phenyl, naphthyl, anthracenyl; branched alkyl groups such as ethylhexyl, isostearyl, octyldodecyl; multibranched alkyl groups such as dimethyl, trimethyl, tetramethyl; linear alkyl groups such as hexyl, octyl, lauryl, myristyl, cetyl, stearyl, arachyl, behenyl; and alkenyl groups such as oleyl and elaidyl.

In one embodiment of the invention, R₁ is preferably cyclohexyl, ethylhexyl, octyl, lauryl, myristyl, stearyl, oleyl, benzyl or phenylethyl.

In one embodiment of the present invention, R₂ is preferably H.

The hydrocarbon group in R₃ is a saturated or unsaturated, linear or branched C2-C21 hydrocarbon group having no cyclic structure, and examples thereof include alkyl groups such as propyl, butyl, pentyl, hexyl, heptyl, octyl, ethylhexyl, and alkenyl groups such as butylene, pentylene, hexylene, heptylene.

In one embodiment of the present invention, R₃ is preferably propylene, butylene, pentylene or hexylene.

The C1-C42 hydrocarbon group in R₄ includes a saturated or unsaturated, linear, branched or cyclic C1-C42 hydrocarbon group, or a C1-C42 hydrocarbon group consisting of a saturated or unsaturated, linear or branched hydrocarbon moiety and a cyclic hydrocarbon moiety, which is typically a fatty acid residue, and examples thereof include cyclic groups such as cyclohexyl, decahydronaphthyl, tetrahydrodicyclopentadiene, sterol, phenyl, naphthyl and anthracel, branched alkyl groups such as ethylhexyl, isostearyl and octyldodecyl, and multibranched alkyl groups such as dimethyl, trimethyl and tetramethyl, linear alkyl groups such as ethyl, propyl, butyl, hexyl, octyl, lauryl, myristyl, cetyl, stearyl, arachyl and behenyl, and alkenyl groups such as oleyl and elaidyl.

In the group represented by formula (II) in R₄ : 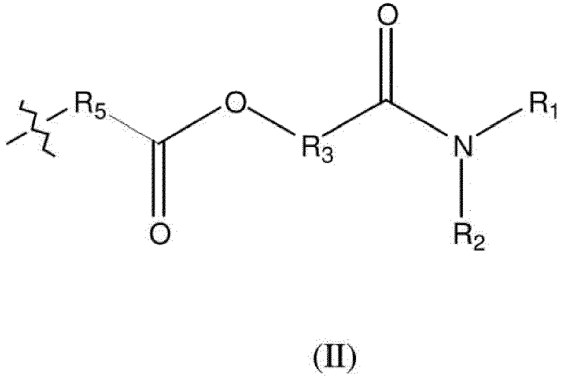 each of R₁ to R₃ may be the same as or different from R₁ to R₃ in formula (I), but are preferably the same, and the compound in which R₁ to R₃ are the same can be expressed by the following formula:

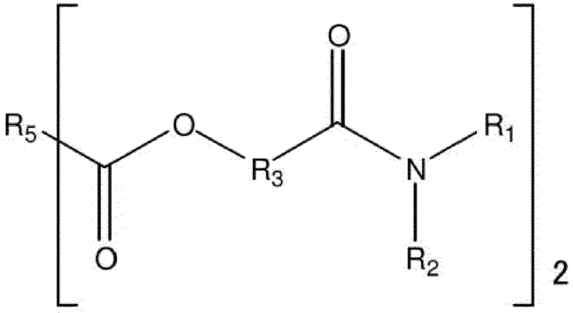

The hydrocarbon group in R₅ includes a saturated or unsaturated, linear, branched or cyclic C2-C42 hydrocarbon group, or a C2-C42 hydrocarbon group consisting of a linear or branched hydrocarbon moiety and a cyclic hydrocarbon moiety, and is typically a residue of carboxylic acid, and examples thereof include alkylene groups such as ethyl, butyl, hexylene, octyl, hydrogenated dimer dilinoleyl, and alkenylene groups such as butene, hexene, etc.

In the present invention, the hydrocarbon group may be substituted with a substituent that does not significantly affect the emulsifying power of the complex, for example, a hydroxy group, a carboxyl group or an aldehyde group. These substituents do not significantly affect the emulsifying power of the complex of the present invention.

Examples of substituted C6-C22 hydrocarbon groups in R₁ and R₂ include hexanol, ethylcyclohexanol, and hexanoic acid.

Examples of substituted C2-C21 hydrocarbon groups in R₃ include hydroxybutyl and butyl ketone.

Examples of substituted C1-C42 hydrocarbon groups in R₄ include hydroxylauryl and hydroxyisostearyl.

Examples of substituted C2-C42 hydrocarbon groups in R₅ include hydroxyethyl and hydroxy hydrogenated dimer dilinoleyl.

In a certain aspect of the present invention, R₅ is a residue of dicarboxylic acid, particularly a residue of dimeric acid.

Dimer acid is a dibasic acid having 36 carbon atoms, which is obtained by polymerizing two molecules in the Diels Alder reaction of an unsaturated fatty acid having 18 carbon atoms such as oleic acid and linoleic acid, etc. A saturated aliphatic dibasic acid having 36 carbon atoms in which the double bond in the dimer acid is reduced by hydrogenation reduction is also called a hydrogenated dimer acid. Dimer acid is usually produced using an unsaturated fatty acid having 18 carbon atoms mainly composed of oleic acid or linoleic acid, and is obtained as a mixture consisting of many compounds including monomer acid, trimer acid, etc. obtained as by-products. The dimer acid used in the present invention may be a mixture of two or more kinds obtained from such a mixture. In one embodiment of the present invention, the dimer acid is preferably hydrogenated dimer acid, and the double bond of dimer acid is preferably completely hydrogenated, but the double bond may remain partially.

In one embodiment of the present invention, a compound of formula (I) wherein:
R₁ is a C10-C22 hydrocarbon group,
R₂ is H,
R₃ is a C3-C12 hydrocarbon group,
R₄ is a C1-C22 hydrocarbon group;
is preferred.

In one embodiment of the invention, the compound of formula (I) is preferably the following compounds:

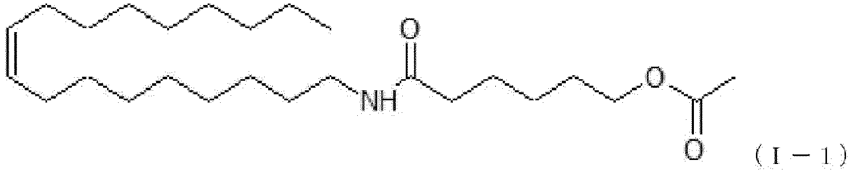

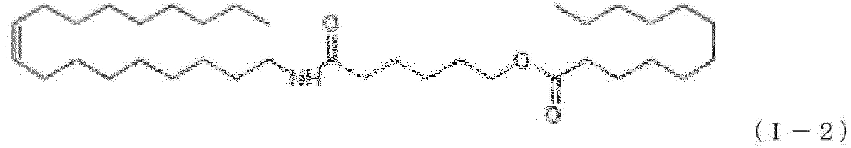

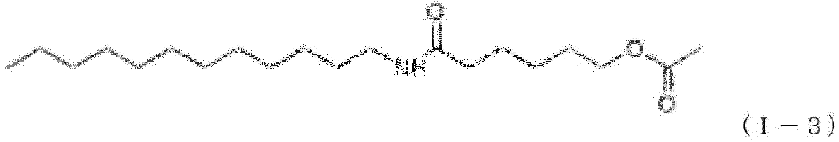

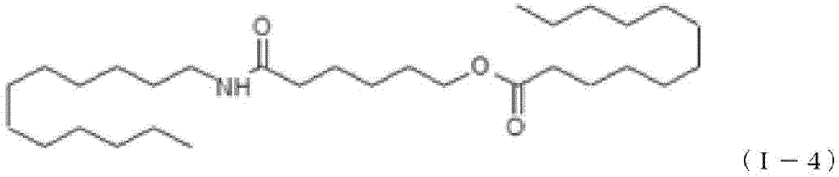

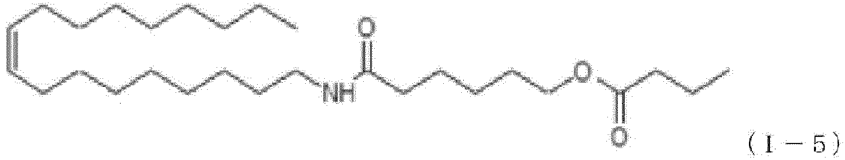

The ester of amide alcohol of formula (I) can be produced by using a known ester production method.

For example, it can be produced by subjecting an amide alcohol represented by the following formula (III): 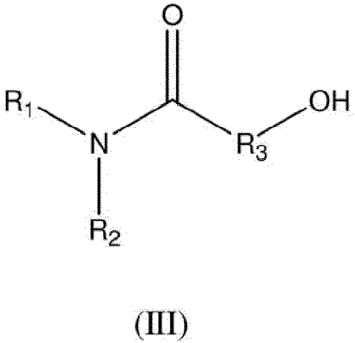 wherein
R₁ to R₃ are the same as in formula (I),
with a C1-C43 fatty acid which may be substituted or a C4-C44 dicarboxylic acid which may be substituted to an esterification reaction by a conventional method.

In one embodiment of the present invention, examples of fatty acid used in the synthesis of the compound of formula (I) include acetic acid, palmitic acid, stearic acid, behenic acid, oleic acid, lauric acid, myristic acid, isostearic acid, etc.

Examples of dicarboxylic acid used in one embodiment of the present invention include succinic acid, adipic acid, hydrogenated dimer dilinoleic acid, and behenic acid dimer, etc.

Specifically, for example, they can be synthesized by the method described in JP A No. 2017-218420.

### Component (B): carboxyl group-containing polymer

The carboxyl group-containing polymer used in the present invention is not particularly limited as long as it is a polymer having a carboxyl group in the molecule. From the viewpoint of providing appropriate emulsifying ability, typically, those having a molecular weight of 500,000 to 3,000,000 and a carboxyl group content of approximately 50 to 70% are preferred.

The carboxyl group-containing polymer becomes water-soluble by neutralization with an alkaline substance, and it is generally used as a thickener.

Examples of carboxyl group-containing polymer include carboxyvinyl polymer, and alkyl-modified carboxyvinyl polymers such as alkyl acrylate/methacrylate copolymer, etc., acrylic polymers such as alkyl acrylate/alkyl methacrylate polyoxyethylene ester copolymer, alkyl acrylate/alkyl itaconate polyoxyethylene ester copolymer, steareth-10 allyl ether/alkyl acrylate copolymer, etc., and non-acrylic polymers such as methyl vinyl ether/maleic anhydride/decadiene copolymer, etc. As the carboxyl group-containing polymer, carboxyvinyl polymers and alkyl-modified carboxyvinyl polymers are particularly preferred.

In one embodiment of the present invention, the carboxyl group-containing polymer is not gelatin.

A carboxyvinyl polymer, also called carbomer (INCI name: Carbomer), is a polymer having a structure represented by the following formula (IV): 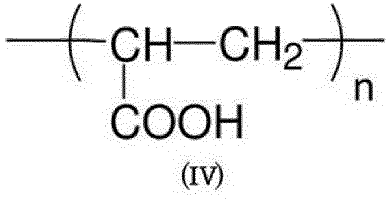 wherein
n is an integer, which is typically from 40 to 100.

Specifically, examples include carboxyvinyl polymers commercially available under the following trade name: Acritamer 934 (Rita Corporation)
Acritamer 940 (Rita Corporation)
Acritamer 941 (Rita Corporation)
Acritamer 990 (Rita Corporation)
Acritamer 501E (Rita Corporation)
Acritamer 504E (Rita Corporation)
Acritamer 505E (Rita Corporation)
AEC Carbomer 940 (A & E Connock (Perfumery & Cosmetics) Ltd.)
Aqupec HV-501 (Sumitomo Seika Chemicals Co., Ltd.)
Aqupec HV-504 (Sumitomo Seika Chemicals Co., Ltd.)
Aqupec HV-505 (Sumitomo Seika Chemicals Co., Ltd.)
Aqupec HV-501E (Sumitomo Seika Chemicals Co., Ltd.)
Aqupec HV-504E (Sumitomo Seika Chemicals Co., Ltd.)
Aqupec HV-505E (Sumitomo Seika Chemicals Co., Ltd.)
Aqupec HV-801E (Sumitomo Seika Chemicals Co., Ltd.)
Aqupec HV-805E (Sumitomo Seika Chemicals Co., Ltd.)
Aqupec HV-505ED (Sumitomo Seika Chemicals Co., Ltd.)
Aqupec HV-801EG (Sumitomo Seika Chemicals Co., Ltd.)
Aqupec HV-805EG (Sumitomo Seika Chemicals Co., Ltd.)

Carbopol Clear Polymer (Lubrizol Advanced Materials, Inc.)
Carbopol ETD 2050 Polymer (Lubrizol Advanced Materials, Inc.)
Carbopol 934 Polymer (Lubrizol Advanced Materials, Inc.)
Carbopol 940 Polymer (Lubrizol Advanced Materials, Inc.)
Carbopol 941 Polymer (Lubrizol Advanced Materials, Inc.)
Carbopol 980 Polymer (Lubrizol Advanced Materials, Inc.)
Carbopol 981 Polymer (Lubrizol Advanced Materials, Inc.)
Carbopol 2984 Polymer (Lubrizol Advanced Materials, Inc.)
Carbopol 5984 Polymer (Lubrizol Advanced Materials, Inc.)
Carbopol Ultrez 10 Polymer (Lubrizol Advanced Materials, Inc.)
Carbopol Ultrez 30 Polymer (Lubrizol Advanced Materials, Inc.)
CustoPoly J 100 (Custom Ingredients, Inc.)
CustoPoly J 300 (Custom Ingredients, Inc.)
CustoPoly J 400 (Custom Ingredients, Inc.)

Easygel DO (3V Sigma USA Inc.)
Flogel 700 (SNF SAS)
Flogel 1000 (SNF SAS)
Junlon PW-110 (Nihon Junyaku Company, Ltd.)
Junlon PW-111 (Nihon Junyaku Company, Ltd.)
Junlon PW-302S (Nihon Junyaku Company, Ltd.)
Polacril 40 (Lehvoss Italia s.r.1.)
Polygel CA (3V Sigma USA Inc.)
Polygel CB (3V Sigma USA Inc.)
Polygel CS (3V Sigma USA Inc.)
Polygel DV (3V Sigma USA Inc.)
Polygel TG (3V Sigma USA Inc.)
SuperGel CE (Sino Lion USA)
Synthalen K (3V Sigma USA Inc.)
Synthalen L (3V Sigma USA Inc.)
Synthalen M (3V Sigma USA Inc.)
Tego Carbomer 134 (Evonik Nutrition & Care GmbH)
Tego Carbomer 140 (Evonik Nutrition & Care GmbH)
Tego Carbomer 141 (Evonik Nutrition & Care GmbH)
Tego Carbomer 340 FD (Evonik Nutrition & Care GmbH).

The alkyl-modified carboxyvinyl polymer is a copolymer of acrylic acid and/or methacrylic acid with alkyl acrylate and/or alkyl methacrylate thereof.

Specific examples of alkyl-modified carboxyvinyl polymer include (acrylates/alkyl acrylates (C10-30)) cross polymer (INCI name: Acrylates/C10-30 Alkyl Acrylates Cross polymer, also called alkyl acrylate/methacrylate copolymer), which is a polymer having a structure represented by the following formula (V): 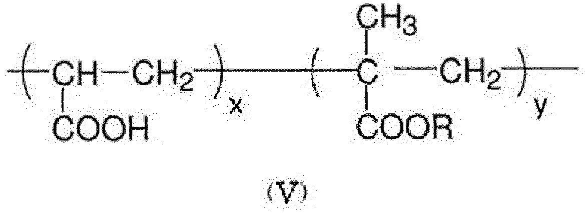 wherein
R is a C10-30 alkyl group,
x and y are integers, each of which can be arbitrarily selected from integers of 1 or more, and typically x + y = 40 to 100, and when y is 2 or more, R may be the same or different.

Specifically, the examples include alkyl-modified carboxyvinyl polymers commercially available under the following trade name:
Acritamer 501ED (Rita Corporation)
Acritamer 505ED (Rita Corporation)
Aqupec HV-701EDR (Sumitomo Seika Chemicals Co., Ltd.)
Aqupec HV-501ER (Sumitomo Seika Chemicals Co., Ltd.)
Aqupec SER W-150C (Sumitomo Seika Chemicals Co., Ltd.)
Aqupec SER W-300C (Sumitomo Seika Chemicals Co., Ltd.)
Carbopol ETD 2020 Polymer (Lubrizol Advanced Materials, Inc.)
Carbopol 1342 Polymer (Lubrizol Advanced Materials, Inc.)
Carbopol 1382 Polymer (Lubrizol Advanced Materials, Inc.)
Carbopol SC 200 (Lubrizol Advanced Materials, Inc.)
Carbopol SC 500 Polymer (Lubrizol Advanced Materials, Inc.)
Carbopol Ultrez 20 Polymer (Lubrizol Advanced Materials, Inc.)
Carbopol Ultrez 21 Polymer (Lubrizol Advanced Materials, Inc.)
Carbopol Xtra-11 Polymer (Lubrizol Advanced Materials, Inc.)
Pemulen EZ-4U Polymeric Emulsifier (Lubrizol Advanced Materials, Inc.)
Pemulen TR-1 Polymer (Lubrizol Advanced Materials, Inc.)
Pemulen TR-2 Polymer (Lubrizol Advanced Materials, Inc.)
Tego Carbomer 341 ER (Evonik Nutrition & Care GmbH)
TEGO Carbomer 750 HD (Evonik Nutrition & Care GmbH).

"Complex" in the present invention means a complex formed from an amide alcohol ester and a carboxyl group-containing polymer.

The complex can be formed by mixing a carboxyl group-containing polymer and an amide alcohol ester. By neutralizing such a mixture with an alkali, a complex having a higher emulsifying ability can be formed.

In one embodiment of the present invention, the complex can be formed by adding an amide alcohol ester to an aqueous solution of a carboxyl group-containing polymer, and then neutralizing with an alkali.

In another embodiment of the present invention, the complex can be formed by adding a carboxyl group-containing polymer to an oil phase containing an amide alcohol ester, and then neutralizing with an alkali.

In yet another embodiment of the present invention, the complex can be formed by adding an aqueous phase comprising a carboxyl group-containing polymer and an alkali to an oil phase containing an amide alcohol ester.

In yet another embodiment of the present invention, the complex can be formed by adding an amide alcohol ester to an aqueous solution comprising a carboxyl group-containing polymer and an alkali.

It is considered that the amide bond portion of the amide alcohol ester and the carbonyl group of the polymer form a hydrogen bond, and a part of the carboxyl group becomes COO⁻ and has hydrophilicity, thereby exerting emulsifying ability.

Thus, while not being bound by any theory, it is believed that the complex of the present invention forms a complex by bonding, more specifically hydrogen bonding of the amide bond moiety of the amide alcohol ester and the carbonyl group of the polymer.

In the present invention, "emulsion" means a composition prepared by emulsifying an oil phase comprising an oily component and an aqueous phase comprising an aqueous component, and includes an O/W emulsion, a W/O emulsion, etc.

In the present invention, the "O/W emulsion" is an oil-in-water emulsion, that is, an emulsion in which an oily component is dispersed in a continuous phase comprising an aqueous component.

In the present invention, the "W/O emulsion" is a water-in-oil emulsion, that is, an emulsion in which an aqueous component is dispersed in a continuous phase comprising an oily component.

The O/W emulsion can be prepared by dispersing an oil phase comprising an amide alcohol ester in an aqueous phase comprising a carboxyl group-containing polymer. When such an emulsion is neutralized with an alkali, a more stable emulsion having finer oil droplets can be prepared.

In one embodiment of the present invention, the O/W emulsion can be prepared by dispersing an oil phase comprising an amide alcohol ester in an aqueous phase comprising a carboxyl group-containing polymer, and neutralizing with an alkali.

In another embodiment of the present invention, the O/W emulsion can be prepared by dispersing an oil phase comprising an amide alcohol ester in an aqueous phase comprising a carboxyl group-containing polymer and an alkali.

The W/O emulsion can be prepared by dispersing an aqueous phase comprising a carboxyl group-containing polymer in an oil phase comprising an amide alcohol ester. When such an emulsion is neutralized with an alkali, a more stable emulsion having finer oil droplets can be prepared.

In one embodiment of the present invention, the W/O emulsion can be prepared by dispersing an aqueous phase comprising a carboxyl group-containing polymer in an oil phase comprising an amide alcohol ester, and neutralizing with an alkali.

In another embodiment of the present invention, the W/O emulsion can be prepared by dispersing an aqueous phase comprising a carboxyl group-containing polymer and an alkali in an oil phase comprising an amide alcohol ester.

In one embodiment of the present invention, emulsions can be used for all purposes, but typically, they can be used for external preparations such as pharmaceuticals, quasi drugs, and cosmetics.

Emulsions of the present invention can be used for various forms of products including pharmaceuticals such as external skin preparation comprising a drug; quasi drugs such as medicated cosmetics; skin care cosmetics such as gel lotion, milky lotion, cream, beauty essence, sunscreen, and daytime moisturizer; makeup cosmetics such as foundation, makeup base, eye shadow, mascara, as well as hair care cosmetics such as hair treatment, etc.

### <Oily component>

The oily component used in the emulsion of the present invention is not particularly limited as long as it is a component generally used for cosmetics and the like; and examples thereof include oil agents such as animal and vegetable fats and oils, hydrocarbon oils, higher fatty acids, higher alcohols, ester oils, silicone oils, which can be used alone or in combination of two or more kinds.

Examples of animal and vegetable fats and oils or hydrogenated animal and vegetable fats and oils include avocado oil, eno oil, olive oil, cacao butter, kaya oil, apricot kernel oil, hydrogenated oil, wheat germ oil, sesame oil, rice germ oil, rice bran oil, sugar cane wax, camellia sinensis leaf oil, safflower oil, shea butter, Chinese tung oil, cinnamon oil, soybean oil, tea seed oil, camellia oil, evening primrose oil, corn oil, rapeseed oil, germ oil, palm oil, palm kernel oil, castor oil, hydrogenated castor oil, sunflower oil, grape oil, jojoba oil, macadamia nut oil, beeswax, cottonseed oil, cotton wax, Japan wax, montan wax, coconut oil, hydrogenated coconut oil, peanut oil, lanolin, liquid lanolin, reduced lanolin, lanolin alcohol, hard lanolin, lanolin acetate, lanolin fatty acid isopropyl ester, hexyl laurate, etc.

Examples of hydrocarbon oils include ozokerite, squalane, squalene, ceresin, paraffin, isoparaffin, paraffin wax, liquid paraffin (mineral oil), pristane, polyisobutylene, polyisobutene, hydrogenated polyisobutene, microcrystalline wax, polyethylene wax, vaseline, etc.

Examples of higher fatty acids include lauric acid, myristic acid, palmitic acid, stearic acid, isostearic acid, oleic acid, behenic acid, undecylenic acid, oleic acid, linoleic acid, linolenic acid, arachidonic acid, eicosapentaenoic acid (EPA), docosahexaenoic acid (DHA), isostearic acid, etc.

Examples of higher alcohols include myristyl alcohol, cetanol, stearyl alcohol, arachidyl alcohol, behenyl alcohol, hydrogenated rapeseed oil alcohol, etc.

Examples of ester oils include, as monoester, isononanoic acid esters such as isononyl isononanoate, isotridecyl isononanoate; 2-ethylhexanoic acids such as cetyl ethylhexanoate, hexyldecyl ethylhexanoate; myristic acid esters such as isopropyl myristate, isocetyl myristate, octyldodecyl myristate; isostearic acid esters such as ethyl isostearate, isopropyl isostearate, hexyldecyl isostearate, isostearyl isostearate, cholesteryl isostearate, phytosteryl isostearate; lactic acid esters such as isostearyl lactate, octyldodecyl lactate; oleic acid esters such as oleyl oleate, phytosteryl oleate, octyldodecyl oleate; neopentanoic acid esters such as isodecyl neopentanoate, isostearyl neopentanoate; palmitic acid esters such as isopropyl palmitate, ethylhexyl palmitate; and others such as octyldodecyl neodecanoate, octyldodecyl ricinoleate, oleyl erucate, octyldodecyl erucate, isopropyl lauroyl sarcosinate.

Examples of diester oils include diisobutyl adipate, diisopropyl adipate, diethylhexyl succinate, neopentyl glycol diisononanoate, neopentyl glycol diethylhexanoate, neopentyl glycol dicaprate, diisostearyl malate, diisopropyl dilinoleate, ethylene glycol dioctanoate, octyldodecyl stearoyl oxystearate, diisopropyl sebacate, di(cholesteryl/octyldodecyl) lauroyl glutamate, di(phytosteryl/octyldodecyl) lauroyl glutamate, etc.

Examples of triester oils include triethylhexanoin, trimethylolpropane triethylhexanoate, glyceryl tri(caprylate/caprate), triisostearin, trimethylolpropane triisostearate, etc.

Examples of tetraester oils include pentaerythrityl tetraethylhexanoate, pentaerythrityl tetraisostearate, etc.

Examples of polyester oils include polyglyceryl fatty acid esters such as polyglyceryl-2 isostearate, polyglyceryl-2 diisostearate, polyglyceryl-2 triisostearate, polyglyceryl-2 tetraisostearate.

Examples of highly viscous ester oils include hydrogenated castor oil isostearate, hydrogenated castor oil dimer dilinoleate, (polyglyceryl-2 isostearate/dimer dilinoleate) copolymer, (phytosteryl/isostearyl/cetyl/stearyl/behenyl) dimer dilinoleate, dimer dilinoleyl
bis(phytosteryl/behenyl/isostearyl) dimer dilinoleate, di(isostearyl/phytosteryl) dimer dilinoleate, dimer dilinoleyl hydrogenated rosin condensate, dimer dilinoleyl diisostearate, dimer dilinoleyl dimer dilinoleate, di(cholesteryl/behenyl/octyldodecyl) lauroyl glutamate, di(octyldodecyl/phytosteryl/behenyl) lauroyl glutamate, myristoyl methylalanine (phytosteryl/decyltetradecyl), etc.

Examples of silicone oils include dimethylpolysiloxane, methyl phenyl polysiloxane, alkyl-modified organopolysiloxane, terminal modified organopolysiloxane, fluorine-modified organopolysiloxane, amodimethicone, amino-modified organopolysiloxane, volatile silicone, alkyl dimethicone, cyclopentasiloxane, etc.

In one embodiment of the present invention, the blending amount of the oily component in the O/W emulsion is not particularly limited; from the viewpoint of usability, it may be 1.0 to 30.0 mass%, preferably 2.0 to 20.0 mass%, and more preferably 5.0 to 15.0 mass%.

In another embodiment of the present invention, the blending amount of the oily component in the W/O emulsion is not particularly limited; from the viewpoint of usability, it may be 30.0 to 80.0 mass%, preferably 40.0 to 70.0 mass%, and more preferably 50.0 to 60.0 mass%.

### <Aqueous component>

Aqueous components used in the emulsion of the present invention are not particularly limited as long as they are components generally used in cosmetics and the like; and examples thereof include water such as purified water, ion exchanged water; and lower alcohols such as BG (1,3-butylene glycol), PG (propylene glycol), glycerin, ethanol, and these can be used alone or in combination of two or more kinds.

In one embodiment of the present invention, the blending amount of the aqueous component in the O/W emulsion is not particularly limited; from the viewpoint of usability, it may be 1.0 to 50.0 mass%, preferably 3.0 to 20.0 mass%, and more preferably 5.0 to 15.0 mass%.

In one embodiment of the present invention, the blending amount of the aqueous component in the W/O emulsion is not particularly limited; from the viewpoint of usability, it may be 10.0 to 70.0 mass%, preferably 15.0 to 65.0 mass%, and more preferably 20.0 to 60.0 mass%.

### <Neutralizing agent>

A neutralizing agent used for preparing the emulsion of the present invention is not particularly limited as long as it is an alkaline component generally used in cosmetics and the like, and examples thereof include potassium hydroxide, triethanolamine, sodium hydroxide, basic amino acids such as L-arginine and L-lysine, 2-amino-2-methyl-1-propanol, etc., and these can be used alone or in combination of two or more kinds.

In addition, the neutralizing agent may be an active component such as tranexamic acid, carnosine.

The blending amount of the neutralizing agent can be appropriately selected depending on the type of the neutralizing agent and the composition of the whole emulsion; it is typically about 0.01 to 1.0 mass%.

### <Surfactant>

In the present specification, the term "surfactant" means a compound having both a hydrophilic group and a hydrophobic group in one molecule, and a surfactant may be appropriately added as necessary to an emulsion.

In one embodiment of the present invention, because the complex of the present invention has an emulsifying ability, preferably, emulsions are substantially free of surfactant. This makes it possible to provide an emulsifier and an emulsion having less stickiness derived from emulsifier, and furthermore, less irritation.

Here, "substantially free of" means that the surfactant is not contained in an amount sufficient for the emulsification of an emulsion. In addition, in the present invention, "substantially free of surfactant" means that it comprises no surfactant at all or comprises a surfactant in an amount that does not emulsify. The amount that does not emulsify can be appropriately determined by a person skilled in the art according to the compositional ratio, for example, in one embodiment it is less than 2.0 mass%, in another embodiment it is less than 0.2 mass%, or less than 0.02 mass%.

In a particular embodiment of the invention, the conditioning composition is an emulsion composition substantially free of surfactant such as cationic surfactant.

### <Other components>

The emulsion of the present invention may comprise any components used in external preparations such as cosmetics, etc.

Examples of these additional components include ultraviolet absorbers such as ethylhexyl methoxycinnamate, hexyl diethylamino hydroxybenzoyl benzoate; thickeners and gelling agents such as dextrin palmitate, xanthan gum; quality maintaining components such as antioxidant, preservative; skin softeners (emollients); medicinal components and active components such as whitening agent, anti-wrinkle agent, antioxidative agent; fragrances, coloring agents such as pigment and dyestuff, and the like.

In one embodiment of the present invention, the oil phase can be oil-gelled with an oil gelling agent in order to improve the stability of the emulsion.

As a component used for oil gelation, an organically modified clay mineral and/or a polymer ester of sugar and fatty acid can be used.

The organically modified clay mineral and the polymer ester of sugar and fatty acid used in the preparation of the emulsion of the present invention are not particularly limited as long as they are components generally used in cosmetics and the like.

Examples of the organically modified clay mineral include those wherein a convertible cation intervening between crystal layers of a water-swellable clay mineral (for example, montmorillonite, saponite, hectorite, bentonite, etc.) is replaced with an organic polar compound or an organic cation (for example, a quaternary ammonium salt type cationic surfactant) .

In the present invention, commercially available organically modified clay minerals can be used; for example, BENTONE 27V (stearalkonium hectorite), BENTONE 27VCG (stearalkonium hectorite), BENTONE 38V (disteardimonium hectorite), BENTONE 38VCG (disteardimonium hectorite), etc. are commercially available from Elementis Specialties.

Organically modified clay minerals are also commercially available as premixes dissolved in silicone oils, ester oils and/or other oils, and premixes may also be used in the present invention; they are commercially available from, for example, Elementis Specialties, under the following trade names.

ENTONE GEL 1002V (cyclopentasiloxane, disteardimonium hectorite, propylene carbonate), BENTONE GEL ABO V (Crambe abyssinica seed oil, stearalkonium hectorite, propylene carbonate), BENTONE GEL CAO V (castor oil, stearalkonium hectorite, propylene carbonate), BENTONE GEL EUG V (octyldodecanol, disteardimonium hectorite, propylene carbonate), BENTONE GTCC V (glyceryl tri(caprylate/caprate), stearalkonium hectorite, propylene carbonate), BENTONE HSO V (glyceryl tri(caprylate/caprate), stearalkonium hectorite, propylene carbonate), BENTONE IHD V (isohexadecane, disteardimonium hectorite, propylene carbonate), BENTONE IPM V (isopropyl myristate, stearalkonium hectorite, propylene carbonate), BENTONE ISD V (isododecane, disteardimonium hectorite, propylene carbonate), BENTONE LOI V (liquid lanolin, isopropyl palmitate, stearalkonium hectorite, propylene carbonate), BENTONE MSO(V) (Meadowfoam seed oil, disteardimonium hectorite, propylene carbonate), BENTONEN GD V (neopentyl glycol diheptanoate, disteardimonium hectorite, propylene carbonate), BENTONE OLV V (olive fruit oil, stearalkonium hectorite, propylene carbonate), BENTONE OMS V ((C11,12) isoparaffin, disteardimonium hectorite, modified alcohol), BENTONE PTIS V (pentaerythrityl tetraisostearate, disteardimonium hectorite, propylene carbonate), BENTONE SS71V (petroleum volatiles, disteardimonium hectorite, propylene carbonate), BENTONE TMF V (methyl trimethicone, disteardimonium hectorite, triethyl citrate), BENTONE TNV ((C12-15) alkyl benzoate, stearalkonium hectorite, propylene carbonate), BENTONE VS-5V(V) (cyclopentasiloxane, disteardimonium hectorite, modified alcohol), BENTONE VS-5PC V(HV) (cyclopentasiloxane, disteardimonium hectorite, propylene carbonate).

The blending amount of the organically modified clay mineral in the emulsion can be appropriately adjusted depending on the type and amount of the oily component, the viscosity required for the emulsion, and the like, and is not particularly limited; it may be 0.5 to 8.0 mass%, preferably 0.7 to 6.0 mass%, more preferably 1.0 to 5.0 mass%.

Examples of the polymer ester of sugar and fatty acid include those commercially available from Chiba Flour Milling Co., Ltd. under the following trade names:
Rheopearl KL2 (dextrin palmitate), Rheopearl TL2 (dextrin palmitate), Rheopearl MKL (dextrin myristate), Rheopearl TT2 [dextrin (palmitate/ethylhexanoate)], Rheopearl 2WX [dextrin (palmitate/hexyldecanoate), Rheopearl ISL2 (inulin stearate), Rheopearl ISK2 (inulin stearate).

The blending amount of the polymer ester of sugar and fatty acid in the emulsion can be appropriately adjusted depending on the type and amount of the oily component, the viscosity required for the emulsion, and the like, and is not particularly limited; it may be 0.5 to 8.0 mass%, preferably 0.7 to 6.0 mass%, more preferably 1.0 to 5.0 mass%.

Furthermore, in one embodiment of the present invention, emulsifying properties can be further improved by blending a polyhydric alcohol such as pentylene glycol and octylene glycol.

Without being bound by any theory, the improvement of emulsifying properties by blending polyhydric alcohol is considered to be due to the formation of a complex by hydrogen bonding between the hydroxy group in the molecule of polyhydric alcohol and the carbonyl group of the amide bond in the molecule of amide alcohol ester.

Accordingly, the present invention also provides a complex formed from an amide alcohol ester, a carboxyl group-containing polymer, a higher fatty acid and a polyhydric alcohol.

It should be noted that pentylene glycol has a moisturizing effect and an antibacterial effect, and can be said to be a preferable component for using the emulsion as an external preparation for cosmetics, etc.

Specifically, pentylene glycol is commercially available under the following trade names.

Examples include Diol PD (Kokyu Alcohol Kogyo Co, Ltd.) and Diol PD-V (Kokyu Alcohol Kogyo Co, Ltd.).

Specifically, octylene glycol is commercially available under the following trade names.

Examples include OD Eight (Kankosha Co., Ltd.).

The blending amount of the polyhydric alcohol can be appropriately adjusted depending on the type and amount of the oily component, the viscosity required for the emulsion, and the like, and is not particularly limited; it may be 0.1 to 10.0 mass%, preferably 0. 5 to 8.0 mass%, more preferably 1.0 to 6.0 mass%.

The blending amount of the component (A) in the emulsion can be appropriately selected depending on the emulsification type, the kind and amount of an oil agent to be used, and the viscosity required, etc.

The blending amount of the component (A) in the O/W emulsion can be appropriately selected depending on the kind and amount of an oil agent to be used, the viscosity required, and the like, and is typically 0.1 to 10. 0 mass%, preferably 0.5 to 8.0 mass%, and more preferably 1.0 to 5.0 mass%.

The blending amount of the component (A) in the W/O emulsion can be appropriately selected depending on the kind and amount of an oil agent to be used, the viscosity required, and the like, and is typically 0.1 to 15. 0 mass%, preferably 0.5 to 10.0 mass%, and more preferably 1.0 to 8.0 mass%.

The blending amount of the component (B) in the emulsion can be appropriately selected depending on the emulsification type, the kind and amount of an oil agent to be used, and the viscosity required, etc.

The blending amount of the component (B) in the O/W emulsion can be appropriately selected depending on the kind and amount of an oil agent to be used, the viscosity required for the emulsion composition, and the like, and is 0.01 to 5.0 mass%, preferably 0.05 to 3.0 mass%, and more preferably 0.1 to 2.0 mass%.

The blending amount of the component (B) in the W/O emulsion can be appropriately selected depending on the kind and amount of an oil agent to be used, the viscosity required, and the like, and is typically 0.01 to 5. 0 mass%, preferably 0.05 to 3.0 mass%, and more preferably 0.1 to 2.0 mass%.

By using the above-mentioned blending amounts, an emulsion composition excellent in usability and stability of emulsion such as oil-in-water type or water-in-oil type of the complex consisting of component A and component B in the present invention can be achieved.

Furthermore, it is possible to provide an emulsifying ability suitable for desired emulsification type (O/W, W/O, etc.) by selecting the ratio between component A and component B.

Here, the pH of the emulsion of the present invention can be appropriately selected, and it is preferably about pH 5.0 to 10.0, and more preferably about pH 5.0 to 8.0. Without being bound by any theory, it is considered that, by making the pH within this range, component A and component B are appropriately hydrogen-bonded to form a complex, and the dissociation of a carboxyl group is moderate, so that excellent emulsifying ability is brought about.

The viscosity of the emulsion of the present invention can be appropriately selected depending on the characteristics of an objective product.

In one embodiment of the present invention, from the viewpoint of obtaining a creamy emulsion, it is preferable to have a viscosity higher than 10,000 mPa·s, and from the viewpoint of obtaining an emulsion in the form of milky lotion, it is preferable to have a viscosity of about 300 to 100,000 mPa·s.

In one embodiment of the present invention, from the viewpoint of using an emulsion as a cosmetic material, the viscosity of the emulsion can be appropriately selected within the range of 300 to 1,000,000 mPa·s.

The present invention provides an agent containing an amide alcohol ester represented by formula (I), wherein the agent is used for emulsification by forming a complex with a carboxyl group-containing polymer.

Furthermore, the present invention also provides an agent containing a carboxyl group-containing polymer, which is used for emulsification by forming a complex with an amide alcohol ester represented by formula (I).

Hereinafter, the present invention will be described in more detail based on examples; however, the present invention is not limited to these examples, and various modifications can be made without departing from the technical idea of the present invention. In the present specification, unless otherwise specified, % means mass%.

### [Examples]

The O/W emulsion compositions shown in the table below were prepared.

### [Table 1]

**Table 1: Formulations of O/W emulsion**

| Material name | Example 1A | Example 1B |
|---|---|---|
| (1) Ion exchanged water | 72.7 | 72.7 |
| (2) Glycerin | 5.0 | 5.0 |
| (3) 1,3-Butylene glycol | 3.0 | 3.0 |
| | | |
| (4) Carbopol ETD2050 (Component B) | 0.3 | 0.3 |
| | | |
| (5) Dimethicone 5cP | 12.0 | 12.0 |
| | | |
| (6) Amide alcohol ester (Component A) | 2.0 | 2.0 |
| | | |
| (7) Alcohol No.20-B | 3.5 | 3.5 |
| | | |
| (8) Potassium hydroxide (10%) | 1.5 | 1.5 |
| Total | 100.0 | 100.0 |
| | | |
| Emulsion particle size (µm) | ∼20 (40) | ∼20 (40) |
| pH | 6.8 | 6.7 |
| Viscosity (mPa·s) S64, 12rpm, 25°C | 11,000 | 6,000 |

Regarding the expression of the emulsion particle size, "∼20 (40)" means that it is on average 20 µm or less, but there are particles with a diameter of about 40 µm scattered in some places. Hereinafter, it is expressed in the same sense.

### (Raw materials)

Material name: Cosmetic ingredient labeling name
Ion Exchanged Water: Water
Glycerin: Glycerin
1,3-Butylene Glycol (Kokyu Alcohol Kogyo Co, Ltd.): Carbopol ETD2050 (Lubrizol Advanced Materials, Inc.): Carboxyvinyl Polymer
Dimethicone 5cp (Momentive Performance Materials Japan LLC): Dimethicone
Alcohol No.20-B (Kokyu Alcohol Kogyo Co, Ltd.): Hydrogenated Rapeseed Oil Alcohol
Potassium Hydroxide (10%): Potassium Hydroxide

As the component A, an amide alcohol ester having the following structure (I-1), which corresponds to formula (I) in which R₁ is unsaturated C18 alkyl (oleyl), R₂ is H, R₃ is C5 alkyl, and R₄ is C1 alkyl, was used.

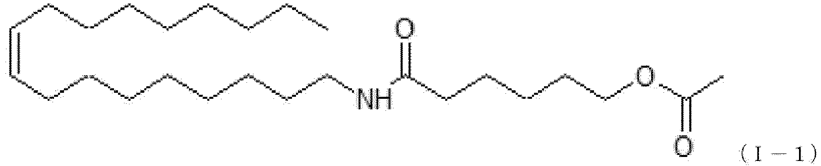

In the present specification, the above amide alcohol ester is also referred to as "amide alcohol ester OLHA".

### (Production methods)

### <Example 1A>

(1) to (4) were uniformly stirred and dissolved at 80°C to obtain an aqueous phase. Meanwhile, (5) to (7) were uniformly stirred and dissolved at 80°C to obtain an oil phase. The oil phase was mixed in the obtained aqueous phase and dissolved uniformly, and stirred with a disperser at 80°C to prepare a mixture. (8) was added with stirring of the mixture to obtain an oil-in-water emulsion composition.

### <Example 1B>

(1) to (4) and (8) were uniformly stirred and dissolved at 80°C to obtain an aqueous phase. Meanwhile, (5) to (7) were uniformly stirred and dissolved at 80°C to obtain an oil phase. The oil phase was mixed in the obtained aqueous phase and dissolved uniformly, and stirred with a disperser at 80°C to obtain an oil-in-water emulsion composition.

### (Evaluation)

### 1. Measurement of emulsion particle size by microscopic observation

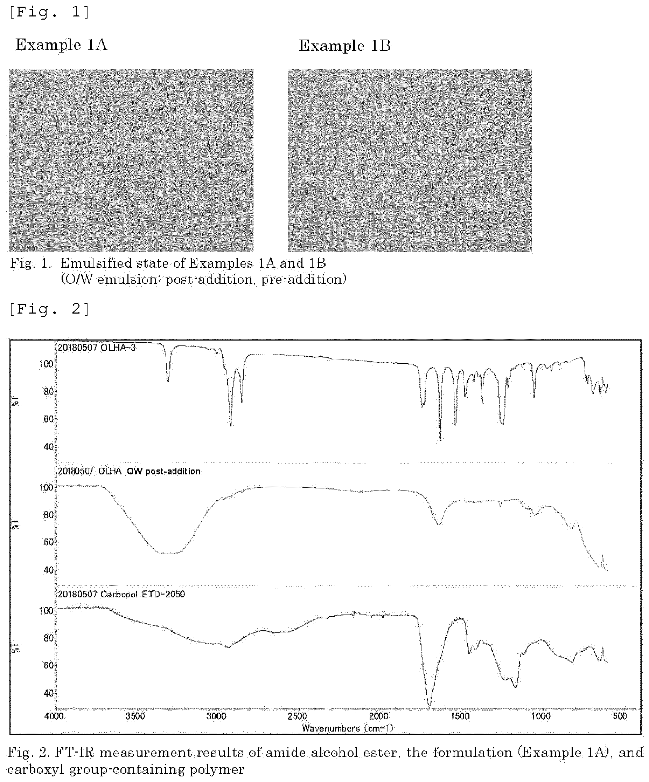

Using "BX-51" manufactured by Olympus Corporation, observation was carried out at 400 times magnification, and emulsion particle size of the emulsions was evaluated. The results are shown in Fig. 1.

The emulsion particle size of the emulsions is on average about 20 µm or less (there rarely are oil droplets of about 40 µm), and it can be said that a highly stable emulsions are obtained.

In addition, the emulsions were stored in a constant temperature bath at 45°C for 1 month and visually observed 1 month later; no change was observed in the emulsified state, and the storage stability was also good.

### 2. Viscosity

Viscosity was measured with Brookfield viscometer: PROGRAMMABLE DV-II + VISCOMETER, using S64 SPINDLE, with a rotation speed of 12 rpm. The viscosity of Example 1A was 11,000 mPa·s, and the viscosity of Example 1B was 6,000 mPa·s. It can be said that emulsions having a viscosity suitable for creamy cosmetic compositions, etc. can be provided.

### 3. pH

pH was measured with a HORIBA pH meter LAQUAact pH/ORP METER D-72. The pH of Example 1A was 6.8 and the pH of Example 1B was 6.7. It can be said that stable emulsions can be provided at a pH suitable for cosmetic compositions.

The formulation in which the composition of Example 1A was naturally dried at 25°C for 2 weeks, as well as the amide alcohol ester and the carbomer were subjected to the measurement of Fourier transform infrared spectrophotometer (hereinafter, FT-IR) using an infrared spectrophotometer (Spectrum One from PerkinElmer Japan, Co., Ltd.) . Measurement results of the amide alcohol ester, the formulation (Example 1A), and the carboxyl group-containing polymer are shown in Fig. 2.

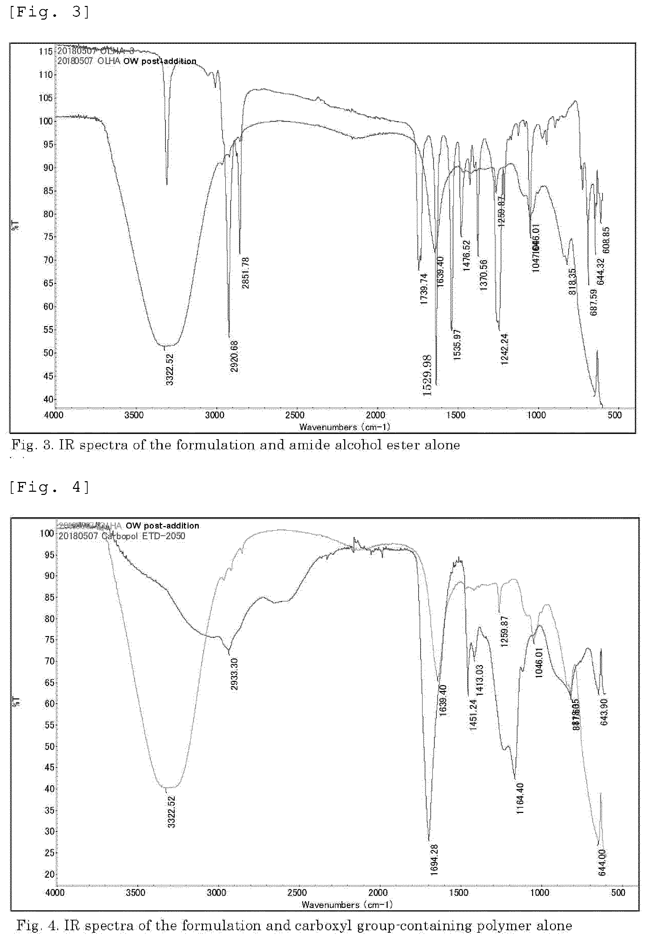

In addition, Fig. 3 shows a diagram in which IR spectra of the formulation and the amide alcohol ester alone are superimposed and compared, and Fig. 4 shows a diagram in which IR spectra of the formulation and the carboxyl group-containing polymer alone are superimposed and compared.

Since O-H stretching (3300 to 2500 cm⁻¹) and O-H vending (1000 to 850 cm⁻¹) by a water molecule mask the absorption of complex formation, natural drying of the emulsion composition was performed to eliminate the absorption by water molecules as much as possible.

From these results, it can be understood that the absorption spectrum of the formulation is different from the superimposed absorption spectra of the amide alcohol ester alone and the carboxyl group-containing polymer alone.

From Fig. 3, in the formulation system, it can be confirmed that N-H bending vibration of the amide alcohol ester at 1535.97 cm⁻¹ disappears, and that C=O stretching vibration at 1631 cm⁻¹ shifts to the high wavelength side.

Furthermore, from Fig. 4, in the formulation system, it can be confirmed that the C=O stretching band of the carboxylic acid of the carboxyl group-containing polymer at 1694.28 cm⁻¹ shifts to the low wavelength side.

This is considered to indicate that COOH, which is a carboxyl group of a part of the carbomer, and the carbonyl group C=O of the amide bond of the amide alcohol ester are bonded by a hydrogen bond to form a complex.

Without being bound by any theory, it is considered that N of the amide bond portion of the amide alcohol and the carboxyl group of the carbomer form a hydrogen bond and exert an emulsifying ability in both non-neutralized and neutralized states.

Without being bound by any theory, it is considered as follows: in the non-neutralized state, the carboxyl group of the carboxyl group-containing polymer is -COOH, and this -COOH forms a hydrogen bond with the carbonyl group of the amide bond of the amide alcohol ester, and the complex is hydrophobic; when this complex is gradually neutralized, a part of the carbomer side chain dissociates into COO- and regains hydrophilicity, and the complex has a hydrophobic part and a hydrophilic part, thereby exhibiting higher emulsifying ability.

However, when pH is in the highly alkaline region of 12.0 or higher, it is considered that dissociation of carboxylate ions proceeds excessively, and the hydrogen bond becomes a hydrogen bond of only the carbonyl group of the amide alcohol ester and the carbonyl group of the carboxyl group ion, and therefore it tends to weaken the complex-forming ability, and the viscosity of the whole preparation reduces and emulsifying performance of the entire agent deteriorates.

### [Table 2]

**Table 2: Evaluation of addition of polyhydric alcohol**

| | | | Example 2A | Example 2B |
|---|---|---|---|---|
| | | Product name | mass% | |
| Oil phase | 1. | Mineral oil | 12.0 | |
| | 2. | Alcohol No.20-B | 3.5 | |
| | 3. | Amide alcohol ester OLHA **(Component A)** | 2.0 | |
| Neutralizing agent | 4. | Potassium hydroxide (10%) | 1.5 | |
| Aqueous phase | **5.** | **Diol PD** | - | **5.0** |
| | 6. | Triol VE | 5.0 | |
| | 7. | Haisugarcane BG | 3.0 | |
| | 8. | Carbopol ETD2050 **(Component B)** | 0.3 | |
| | 9. | Purified water | 72.7 | 67.7 |
| Emulsion particle size (µm) | | | ∼30 (50) | ∼10 (20) |

### (Raw materials used)

Product name: Cosmetic ingredient labeling name
Diol PD (Kokyu Alcohol Kogyo Co, Ltd.): Pentylene Glycol
Triol VE (Kokyu Alcohol Kogyo Co, Ltd.): Glycerin
Haisugarcane BG (Kokyu Alcohol Kogyo Co, Ltd.) : Butylene Glycol

### (Production method)

(1) Each of oil phase and aqueous phase is uniformly dissolved at 80°C.
(2) While stirring the aqueous phase with a homomixer, the oil phase is added (800 rpm).
(3) After emulsification, the mixture is stirred with a homomixer (2000 rpm, 3 min).
(4) Raw material 4 is added and further stirred (2000 rpm, 3 min) .
(5) While stirring by hand, the mixture is slowly cooled to room temperature.

### (Evaluation)

The emulsion particle size was evaluated by the same method as in Example 1.

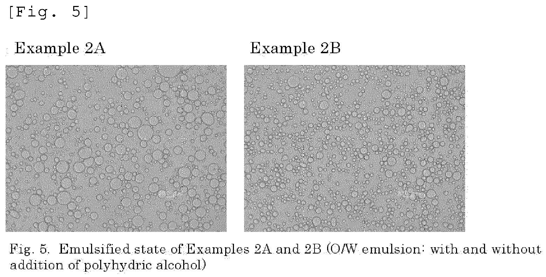

The results are shown in Fig. 5.

It can be understood that by adding pentylene glycol (Diol PD), the emulsifying properties are improved and an emulsion composition having a smaller particle size can be obtained.

### <Example 3>

### [Table 3]

**Table 3: Evaluation of different oil agents and amide alcohol esters**

| | | Product name | mass% |
|---|---|---|---|
| Oil phase | **1.** | **Oil agent** | **12.0** |
| | 2. | Alcohol No.20-B | 3.5 |
| | **3.** | **Amide alcohol ester (Component A)** | **2.0** |
| Neutralizing agent | 4. | Potassium hydroxide (10%) | 1.5 |
| Aqueous phase | 5. | Diol PD | 5.0 |
| | 6. | Triol VE | 5.0 |
| | 7. | Haisugarcane BG | 3.0 |
| | 8. | Carbopol ETD2050 **(Component B)** | 0.3 |
| | 9. | Purified water | 67.7 |

### (Raw materials used)

As an oil agent in the above table, any of the following was used:
- Mineral oil (trade name : Hicall K-230, Kaneda Co., Ltd.)
- Dimethicone 5cP (trade name: Element14 PDMS 5-JC, Momentive Performance Materials Japan LLC)
- Isononyl isononanoate (trade name: KAK 99, Kokyu Alcohol Kogyo Co, Ltd.)

As the amide alcohol ester in the above table, any of the following was used:
- Amid alcohol ester OLHA represented by the above formula (I-1)
- Amide alcohol ester having the following structure (1-2), which corresponds to formula (I) in which R₁ is unsaturated C18 alkyl (oleyl), R₂ is H, R₃ is C5 alkyl, and R₄ is C11 alkyl (hereinafter, also referred to as "amide alcohol ester OLHL"),
- Amide alcohol ester having the following structure (I-3), which corresponds to formula (I) in which R₁ is saturated C12 alkyl, R₂ is H, R₃ is C5 alkyl, and R₄ is C1 alkyl (hereinafter, also referred to as "amide alcohol ester LHA"),
- Amide alcohol ester having the following structure (I-4), which corresponds to formula (I) in which R₁ is saturated C12 alkyl, R₂ is H, R₃ is C5 alkyl, and R₄ is C11 alkyl (hereinafter, also referred to as "amide alcohol ester LHL"),
- Amide alcohol ester having the following structure (1-5), which corresponds to formula (I) in which R₁ is unsaturated C18 alkyl (oleyl), R₂ is H, R₃ is C5 alkyl, and R₄ is C3 alkyl (hereinafter, also referred to as "amide alcohol ester OLHB").

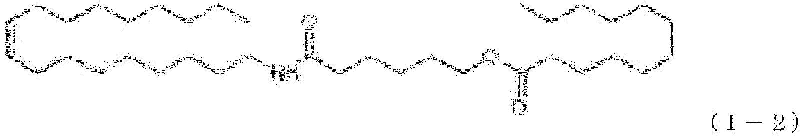

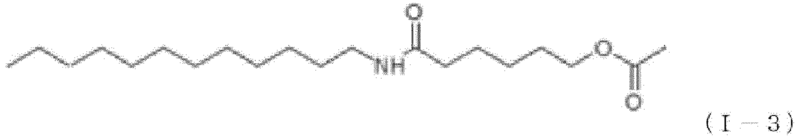

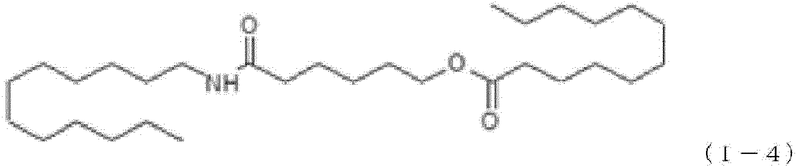

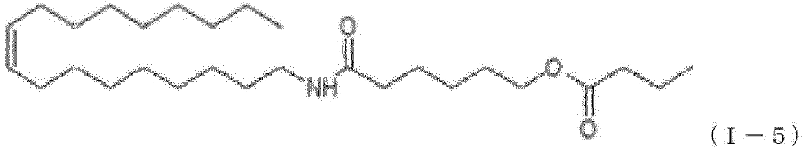

### (Production method)

### <Pre-addition of neutralizing agent>

(1) Raw material 4 is added to raw materials 1 to 3 and the mixture is moistened.
(2) Each of raw materials 6 to 9 is uniformly dissolved at 80°C.
(3) The oil phase is added while stirring the aqueous phase with a homomixer (800 rpm).
(4) After emulsification, the mixture is stirred with a homomixer (3000 rpm, 6 min)
(5) While stirring by hand, the mixture is slowly cooled to room temperature.

### <Post-addition of neutralizing agent>

(1) Each of the oil phase and aqueous phase is uniformly dissolved at 80°C.
(2) While stirring the aqueous phase with a homomixer, the oil phase is added (800 rpm).
(3) After emulsification, the mixture is stirred with a homomixer (2000 rpm, 3 min).
(4) Raw material 4 is added and further stirred (2000 rpm, 3 min) .
(5) While stirring by hand, the mixture is slowly cooled to room temperature.

### (Evaluation)

The emulsion particle size was evaluated by the same method as in Example 1.

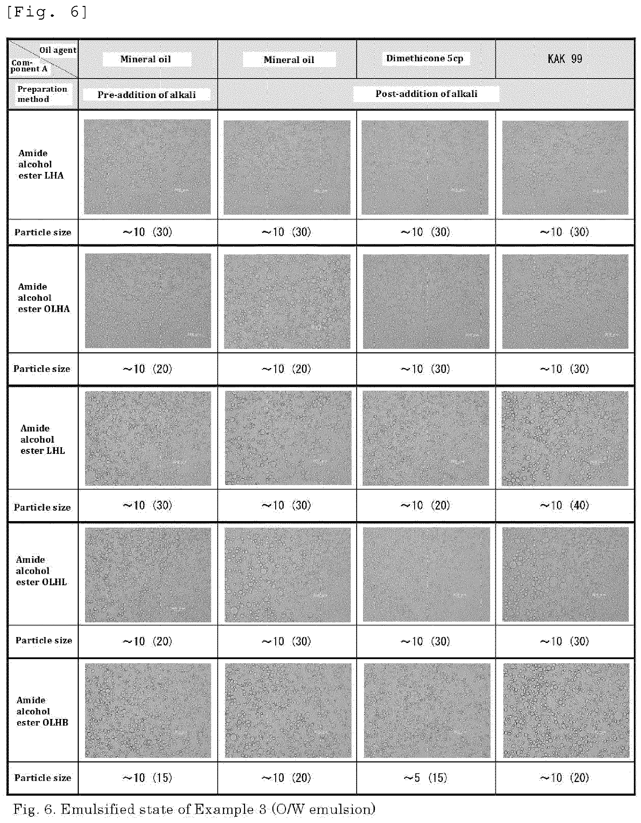

The results are shown in Fig. 6.

Emulsifying property was confirmed in the emulsification of various oils.

The W/O emulsion composition described in the table below was prepared.

### [Table 4]

**Table 4: Composition of W/O emulsion composition**

| Material name | Example 4 |
|---|---|
| (1) Ion exchanged water | 40.45 |
| (2) Glycerin | 5.0 |
| (3) 1,3-Butylene glycol | 3.0 |
| (4) Diol PD | 2.0 |
| | |
| (5) PEMULEN TR-2 **(Component B)** | 0.2 |
| | |
| (6) Squalane | 35.1 |
| | |
| (7) Amide alcohol ester OLHA **(Component A)** | 6.00 |
| (8) BENTONE GEL ISD V | 3.5 |
| (9) Rheopearl KL2 | 3.0 |
| (10) Rheopearl WX | 1.5 |
| (11) Potassium hydroxide (10%) | 0.25 |
| Total | 100.0 |
| | |
| Emulsion particle size (µm) | ∼30 |
| Viscosity (mPa·s) (No.64, 1.5 rpm) | 315,000 |

### (Raw materials used)

Material name: Cosmetic ingredient labeling name
1,3-Butylene glycol (Kokyu Alcohol Kogyo Co, Ltd.): Butylene Glycol
PEMULEN TR-2 (Lubrizol Advanced Materials, Inc.): Alkyl-Modified Carboxyvinyl Polymer
Squalane: Squalene
BENTONE GEL ISD V (Elementis Specialties): Isohexadecane, Disteardimonium Hectorite, Propylene Carbonate
Rheopearl Kl2 (Chiba Flour Milling Co., Ltd.): Dextrin Palmitate
Rheopearl WX (Chiba Flour Milling Co., Ltd.): Dextrin (Palmitate/Hexyldecanoate)

### (Production method)

(1) to (5) were uniformly stirred and dissolved at 80°C to obtain an aqueous phase. Meanwhile, (6) to (10) were uniformly stirred and dissolved at 80°C to obtain an oil phase. The aqueous phase was mixed in the obtained oil phase and uniformly dissolved, and stirred with a disperser at 80°C to prepare a mixture. While stirring the mixture, (11) was added to obtain a water-in-oil emulsion composition.

A W/O emulsion was obtained from the carboxyl group-containing polymer and the amide alcohol. The emulsified state is shown in Fig. 5.

### (Evaluation)

### 1. Measurement of emulsion particle size by microscopic observation

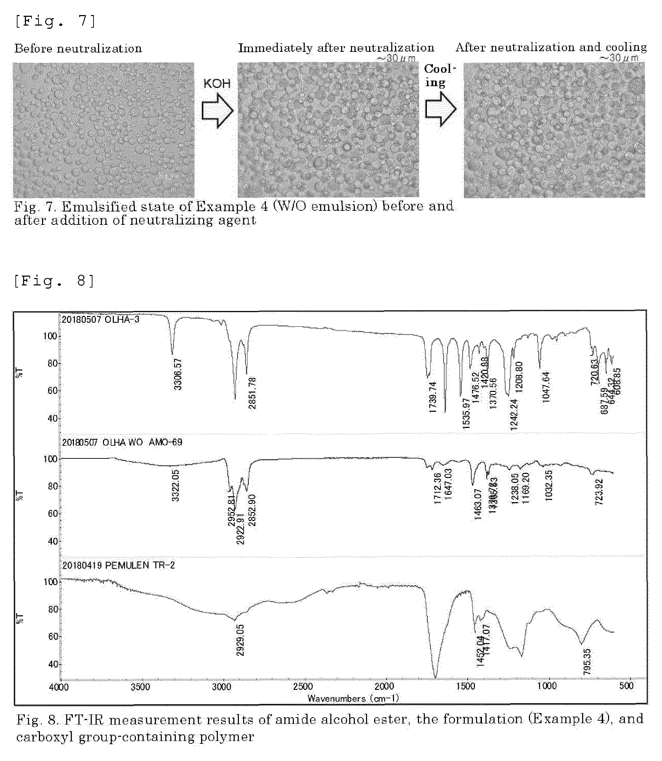

The emulsion particle size of the emulsion before neutralization, after neutralization, and after neutralization & cooling was measured by the same method as in Example 1. The results are shown in Fig. 7.

The emulsion particle size of the emulsion is about 30 µm or less on average, and it can be said that a very stable emulsion is obtained.

### 2. Viscosity

Viscosity was measured by the same method as in Example 1 except that the rotation speed was set to 1. 5 rpm in accordance with the viscosity.

It can be said that an emulsion having a viscosity suitable for creamy cosmetic compositions, etc. can be provided.

The formulation in which the composition of Example 4 was naturally dried at 25°C for 2 weeks, as well as the amide alcohol ester and the carbomer were subjected to the measurement of Fourier transform infrared spectrophotometer (hereinafter, FT-IR) using an infrared spectrophotometer (Spectrum One from PerkinElmer Japan, Co., Ltd.). Measurement results of the amide alcohol ester, the formulation (Example 4), and the carboxyl group-containing polymer are shown in Fig. 8.

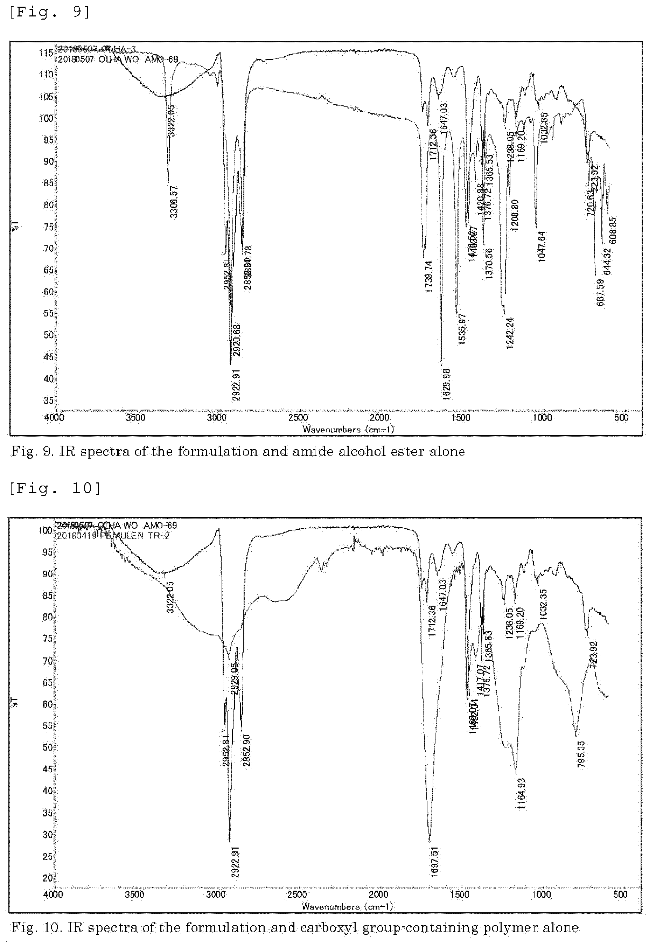

In addition, Fig. 9 shows a diagram in which IR spectra of the agent and the amide alcohol ester alone are superimposed and compared, and Fig. 10 shows a diagram in which IR spectra of the agent and the carboxyl group-containing polymer alone are superimposed and compared.

Since O-H stretching vibration (3300 to 2500 cm⁻¹) and O-H vending vibration (1000 to 850 cm⁻¹) by a water molecule mask the absorption of complex formation, natural drying of the emulsion composition was performed to eliminate the absorption by water molecules as much as possible.

From these results, it can be understood that the absorption spectrum of the agent is different from the superimposed absorption spectra of the amide alcohol ester alone and the carboxyl group-containing polymer alone.

From Fig. 9, in the formulation system, it can be confirmed that N-H bending vibration of the amide alcohol ester at 1535.97 cm⁻¹ disappears, and that C=O stretching vibration at 1629.98 cm⁻¹ shifts to the high wavelength side.

Furthermore, from Fig. 10, in the agent system, it can be confirmed that the C=O stretching band of the carboxylic acid of the carboxyl group-containing polymer at 1697.51 cm⁻¹ shifts to the high wavelength side.

This is considered to indicate that COOH, which is a carboxyl group of a part of the carbomer, and the carbonyl group C=O of the amide bond of the amide alcohol ester are bonded by a hydrogen bond to form a complex.

Without being bound by any theory, it is considered that N of the amide bond portion of the amide alcohol and the carboxyl group of the carbomer form a hydrogen bond and exert an emulsifying ability in both non-neutralized and neutralized states.

Without being bound by any theory, it is considered as follows: in the non-neutralized state, the carboxyl group of the carboxyl group-containing polymer is -COOH, and this -COOH forms a hydrogen bond with the carbonyl group of the amide bond of the amide alcohol ester, and the complex is hydrophobic; when this complex is gradually neutralized, a part of the carbomer side chain dissociates into COO- and regains hydrophilicity, and the complex has a hydrophobic part and a hydrophilic part, thereby exhibiting higher emulsifying ability.

### [Table 5]

**Table 5: Evaluation of different amide alcohol esters**

| | | 5A | 5B | 5C | 5D | 5E | 5F |
|---|---|---|---|---|---|---|---|
| | | Post-addition | Pre-addition | Post-addition | Pre-addition | Post-addition | Pre-addition |
| Material name | | | | | | | |
| (1) Ion exchanged water | | 34.55 | | 34.55 | | 35.55 | |
| (2) Glycerin | | 5.0 | | 5.0 | | 5.0 | |
| (3) 1,3-Butylene glycol | | 3.0 | | 3.0 | | 3.0 | |
| (4) Diol PD | | 2.0 | | 2.0 | | 2.0 | |
| | | | | | | | |
| (5) PEMULEN TR-2 **(Comp. B)** | | 0.2 | | 0.2 | | 0.2 | |
| | | | | | | | |
| (6) Squalane | | 35.1 | | 35.1 | | 36.1 | |
| | | | | | | | |
| (7) Amide alcohol ester **(Comp. A)** | OLHL | 6.00 | | - | | - | |
| | LHA | - | | 6.00 | | - | |
| | OLHB | - | | - | | 6.00 | |
| (8) Isostearic acid EX | | 5.9 | | 5. 9 | | 5. 9 | |
| (9) BENTONE GEL ISD V | | 3.5 | | 3.5 | | 3.5 | |
| (10) Rheopearl KL2 | | 3.0 | | 3.0 | | 1.5 | |
| (11) Rheopearl WX | | 1.5 | | 1.5 | | 1.0 | |
| | | | | | | | |
| (12) Potassium hydroxide (10% aq. solution) | | 0.25 | | 0.25 | | 0.25 | |
| Total | | 100.0 | | 100.0 | | 100.0 | |
| Emulsion particle size (µm) | | ∼30 | ∼20 | ∼ 30 (50) | ∼ 30 (50) | ∼50 | ∼70 |
| Viscosity (mPa·s) (No. 64, 0.3 rpm) | | 1,860,0 00 | 454,00 0 | 1,650,0 00 | 1,020,0 00 | 926,000 | 1,040, 000 |

### (Raw material)

Isostearic Acid EX (Kokyu Alcohol Kogyo Co, Ltd.) : Isostearic Acid
(Production method 1: Post-addition of neutralizing agent, Examples 5A, 5C, 5E)

(1) to (5) were uniformly stirred and dissolved at 80°C to obtain an aqueous phase. Meanwhile, (6) to (11) were uniformly stirred and dissolved at 80°C to obtain an oil phase. The aqueous phase was mixed in the obtained oil phase and uniformly dissolved, and stirred with a disperser at 80°C to prepare a mixture. While stirring the mixture, (12) was added to obtain a water-in-oil emulsion composition. (Production method 2: pre-addition of neutralizing agent, Example 5B, 5D, 5F)

(1) to (5) and (12) were uniformly stirred and dissolved at 80°C to obtain an aqueous phase. Meanwhile, (6) to (11) were uniformly stirred and dissolved at 80°C to obtain an oil phase. The aqueous phase was mixed in the obtained oil phase and uniformly dissolved, and stirred with a disperser at 80°C to prepare a mixture.

### (Evaluation)

### 1. Measurement of emulsion particle size by microscopic observation

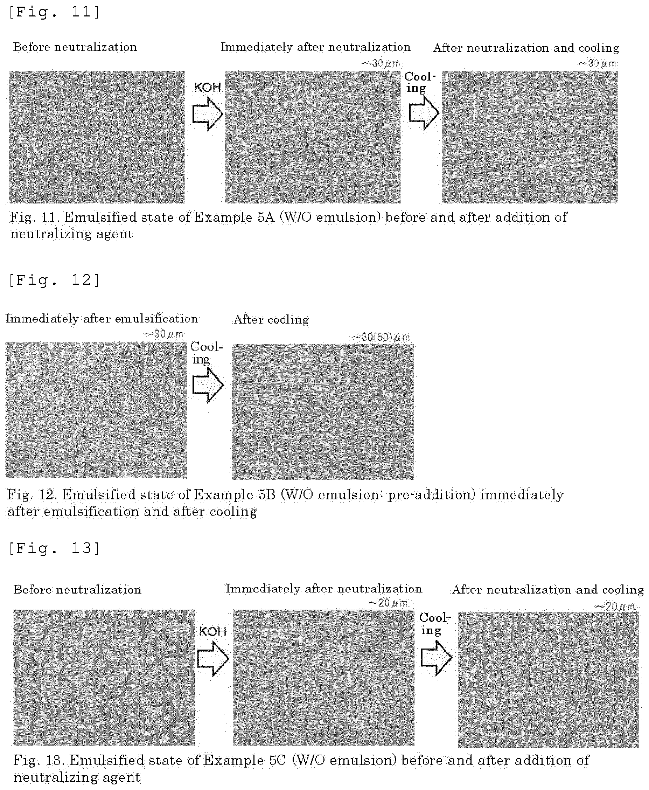

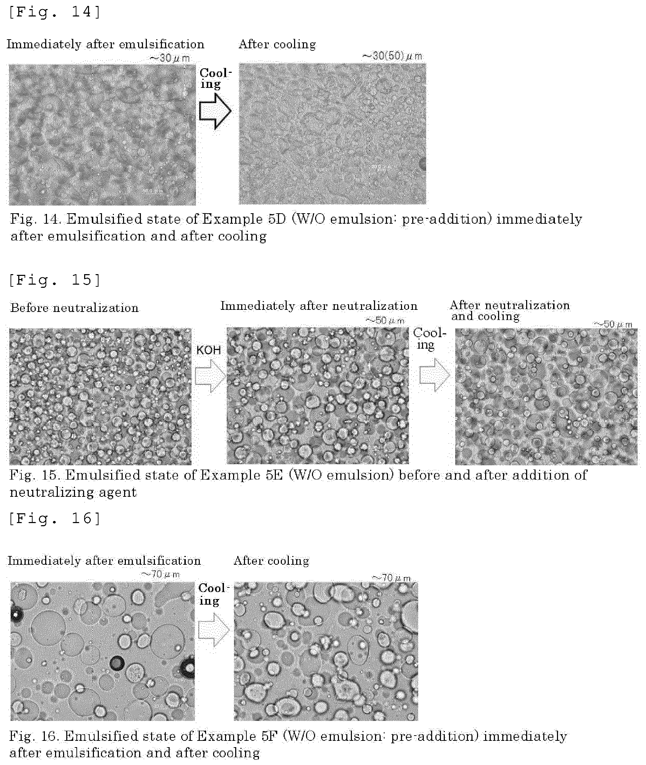

For the production method 1, the emulsion particle size of the emulsion before neutralization, after neutralization, and after neutralization & cooling was measured; and for the production method 2, emulsion particle size of the emulsion immediately after emulsification and after cooling was measured, with the same method as in Example 1. The results of Examples 5A to 5F are shown in Figs. 11 to 16, respectively.

It can be said that stable emulsions are obtained.

### 2. Viscosity

Viscosity was measured by the same method as in Example 1 except that the rotation speed was set to 0. 3 rpm in accordance with the viscosity.

It can be said that an emulsion having a viscosity suitable for creamy cosmetic compositions, etc. can be provided.

Examples of O/W emulsion cosmetic compositions using the complex of the present invention are shown below.

### [Example 6] Hair treatment lotion

**[Table 6]**

| | | Blending amount (%) |
|---|---|---|
| (1) | Propylene glycol | 2.0 |
| (2) | Glycerin | 1.0 |
| (3) | Alkyl-modified carboxyvinyl polymer **(Component B)** Trade name: Pemulen TR-1, Lubrizol Advanced Materials, Inc. | 0.1 |
| (4) | Amide alcohol ester OLHA of structural formula (I) **(Component A)** | 0.5 |
| (5) | Methyl phenyl polysiloxane | 1.0 |
| (6) | Keratin hydrolyzate Trade name: Promois WK-H, Seiwa Kasei Co., Ltd. | 1.0 |
| (7) | Methylparaben | 0.1 |
| (8) | Octyl methoxycinnamate | 0.01 |
| (9) | Ethanol | 5.0 |
| (10) | Potassium hydroxide | 0.15 |
| (11) | Ion exchanged water | Balance |
| (12) | Fragrance | 0.01 |

### <Production method>

(1) to (3), (6), (7) and (11) are heated to 80°C and uniformly dissolved (aqueous phase). Meanwhile, (4), (5) and (8) are uniformly dissolved at 80°C to obtain an oil phase.

While adding the oil phase to the aqueous phase, these are stirred with a disperser. Then, a solution prepared with (10) and a part of (11) is added and emulsified. Upon completion of the emulsification, the mixed solution of (9) and (12) is added and cooled to normal temperature, to obtain a targeted hair treatment lotion which is an O/W emulsion having pH 6.2.

### [Example 7] Milky lotion

**[Table 7]**

| | | Blending amount (%) |
|---|---|---|
| (1) | Dimethicone 5cs | 10.0 |
| (2) | Squalane | 10.0 |
| (3) | Olefin oligomer | 6.0 |
| (4) | Isotridecyl isononanoate Trade name: KAK 139, Kokyu Alcohol Kogyo Co., Ltd. | 5.0 |
| (5) | Amide alcohol ester LHA of structural formula (I) **(Component A)** | 1.5 |
| (6) | Fragrance | Suitable amount |
| (7) | Dipropylene glycol | 1.0 |
| (8) | 1,3-Butylene glycol | 4.0 |
| (9) | Glycerin | 6.0 |
| (10) | Carboxyvinyl polymer (Component B) Trade name: Carbopol 981 Polymer, Lubrizol Advanced Materials, Inc. | 0.1 |
| (11) | Alkyl-modified carboxyvinyl polymer **(Component B)** Trade name: Pemulen TR-2, Lubrizol Advanced Materials, Inc. | 0.1 |
| (12) | Sodium hydroxide | Suitable amount |
| (13) | Acetylated hyaluronic acid | 0.01 |
| (14) | Polymethacryloyl ethyl phosphorylcholine derivative Trade name: LIPIDURE-PMB (Ph10), NOF Corporation | 0.1 |
| (15) | Equisetum arvense extract | 0.1 |
| (16) | Hamamelis virginiana (witch hazel) leaf extract | 0.1 |
| (17) | Ethanol | 5.0 |
| (18) | Phenoxyethanol | 0.3 |
| (19) | Ion exchanged water | Balance |
| (20) | Polyvinyl alcohol | 0.3 |

### <Production method>

(7) to (11) and (13) to (16) are uniformly dissolved at 80°C (aqueous phase). Meanwhile, (1) to (5) are uniformly dissolved at 80°C, added to the aqueous phase, and stirred with a homomixer at 80°C. Next, an aqueous solution of (12) dissolved in a part of (19) is added and emulsified again with a homomixer. After completion of the emulsification, the mixed solution of (6) and (7) is added and cooled to normal temperature to obtain a targeted milky lotion having pH 6.8.

Examples of W/O emulsion cosmetic compositions using the complex of the present invention are shown below.

### [Example 8] Hair treatment cream

**[Table 8]**

| | | Blending amount (%) |
|---|---|---|
| (1) | Propylene glycol | 2.0 |
| (2) | Glycerin | 1.0 |
| (3) | Alkyl-modified carboxyvinyl polymer **(Component B)** Trade name: Pemulen TR-1, Lubrizol Advanced Materials, Inc. | 0.1 |
| (4) | Amide alcohol ester (OLHA) of structural formula (I) **(Component A)** | 3.5 |
| (5) | Squalane (Trade name: Olive squalane, Kokyu Alcohol Kogyo Co., Ltd.) | 35.0 |
| (6) | Methyl phenyl polysiloxane | 1.0 |
| (7) | Organically modified clay mineral premix (Trade name: BENTONE IHD V (isohexadecane, disteardimonium hectorite, propylene carbonate), Elementis Specialties) | 4.0 |
| (8) | Isostearic acid (Trade name: HAIMARIC MKH(R), Kokyu Alcohol Kogyo Co., Ltd.) | 3.0 |
| (9) | Dextrin palmitate (Trade name: Rheopearl KL2, Rheopearl TL2, Chiba Flour Milling Co, Ltd.) | 3.0 |
| (10) | Keratin hydrolyzate Trade name: Promois WK-H, Seiwa Kasei Co., Ltd. | 1.0 |
| (11) | Methylparaben | 0.1 |
| (12) | Octyl methoxycinnamate | 0.01 |
| (13) | Sodium hydroxide (10% aqueous solution) | 0.1 |
| (14) | Ion exchanged water | Balance |
| (15) | Fragrance | 0.1 |

### <Production method>

(4) to (9) and (12) are uniformly mixed at 80°C (oil phase) .

Meanwhile, (1) to (3), (10), (11) and (14) are uniformly mixed at 80°C (aqueous phase).

While adding the aqueous phase to the oil phase, the mixture is stirred with a disperser.

Next, (13) is added and emulsified. Upon completion of the emulsification, (15) is added and the mixture is cooled to normal temperature to obtain a targeted viscosity of 1,980,000 mPa·s.

### [Example 9] W/O emulsion foundation

**[Table 9]**

| | | Blendin g amount (%) |
|---|---|---|
| (1) | Silicone-coated titanium oxide | 18.0 |
| (2) | Silicone-coated iron oxide (red) | 0.3 |
| (3) | Silicone-coated iron oxide (black) | 0.015 |
| (4) | Silicone-coated iron oxide (yellow) | 1.2 |
| (5) | Alkyl-modified carboxyvinyl polymer **(Component B)** Trade name: Pemulen TR-2, Lubrizol Advanced Materials, Inc. | 0.2 |
| (6) | Decamethylcyclopentasiloxane | 35.0 |
| (7) | Trimethylsiloxysilicate/Decamethylcyclopentasilox ane solution Trade name: X-21-5250, Shin-Etsu Chemical Co., Ltd. | 5.0 |
| (8) | Amide alcohol ester (OLHL) of structural formula (I) | 3.0 |
| | **(Component A)** | |
| (9) | Sodium hydroxide | 0.1 |
| (10 ) | Ion exchanged water | Balance |
| (11 ) | Disteardimonium hectorite (Trade name: BENTON 38V, Elementis Specialties) | 5.5 |
| (12 ) | Isostearic acid (Trade name: Isostearic acid EX, Kokyu Alcohol Kogyo Co., Ltd.) | 5.0 |
| (13 ) | Dextrin palmitate/hexyldecanoate (Trade name: Rheopearl WX, Chiba Flour Milling Co., Ltd. ) | 3.5 |
| (14 ) | Fragrance | Suitabl e amount |
| (15 ) | Pentylene glycol (Trade name: Diol PD-V, Kokyu Alcohol Kogyo Co., Ltd. ) | 2.00 |

### <Production method>

(1) to (4), (6) to (8), and (11) to (14) are uniformly dispersed at 80°C (oil phase).

Meanwhile, an aqueous phase of (5), (9) and (15) is uniformly dissolved and mixed at 80°C, and the mixture is stirred with a disperser while slowly being added to the previously prepared oil phase.

Furthermore, a solution prepared with (9) and a part of (10) is added and emulsified. Upon completion of the emulsification, the mixture is cooled to normal temperature to obtain a targeted W/O emulsion foundation having a viscosity of 715,000 mPa·s.

### [Example 10] Emollient cream

**[Table 10]**

| | | Blending amount (%) |
|---|---|---|
| (1) | Hydrogenated polyisobutene | 3.0 |
| (2) | Liquid paraffin | 3.0 |
| (3) | Isostearyl neopentanoate Trade name: Neolight 180P, Kokyu Alcohol Kogyo Co. , Ltd. | 6.0 |
| (4) (5) | Decamethylcyclopentasiloxane (Dimethicone/Phenylvinyl | 5.0 |
| | dimethicone)crosspolymer /Diphenylsiloxy phenyl trimethicone mixture (Trade name: KSG-18A, Shin-Etsu Chemical Co., Ltd. ) | 0.5 |
| (6) | Fragrance | Suitable amount |
| (7) | Amide alcohol ester (LHA) of structural formula (I) **(Component A)** | 6.0 |
| (8) | Ethylparaben | 0.1 |
| (9) | Butylparaben | 0.1 |
| (10) | Tocopherol | 0.5 |
| (11) | Alkyl-modified carboxyvinyl polymer **(Component B)** (Trade name: Pemulen TR-2, Lubrizol Advanced Materials, Inc.) | 0.15 |
| (12) | Carboxyvinyl polymer **(Component B)** (Trade name: Synthalen L, 3V Sigma USA Inc.) | 0.15 |
| (13) | Polyethylene glycol 20000 | 1.0 |
| (14) | Crataegus cuneata fruit extract | 0.1 |
| (15) | Syzygium jambos leaf extract | 0.1 |
| (16) | Aloe extract | 0.1 |
| (17) | Sanguisorba officinalis root extract | 0.1 |
| (18) | Eugenia Caryophyllus (clove) flower extract | 0.1 |
| (19) | Houttuynia cordata extract | 0.1 |
| (20) | Althaea officinalis root extract | 0.1 |
| (21) | Lithospermum officinale root extract | 0.1 |
| (22) | 1,3-Butylene glycol | 3.0 |
| (23) | Glycerin | 5.0 |
| (24) | Pentylene glycol (Trade name: Diol PD-V, Kokyu Alcohol Kogyo Co., Ltd. ) | 2.0 |
| (25) | Ion exchanged water | Balance |
| (26) | Potassium hydroxide | Suitable amount |
| (27) | Organically modified clay mineral premix (Trade name: BENTONE ISD V (isododecane, disteardimonium hectorite, propylene carbonate), Elementis Specialties) | 4.0 |
| (28) | Dextrin palmitate/Ethylhexanoate (Trade name: Rheopearl TT2, Chiba Flour Milling Co., Ltd. ) | 3.0 |

### <Production method>

(1) to (10) are heated to 80°C and uniformly mixed (oil phase).

Meanwhile, (11) to (25), (27) and (28) are uniformly mixed at 80°C (aqueous phase).

While gradually adding the aqueous phase to the previously prepared oil phase at 80°C, the mixture is stirred with a disperser.

Furthermore, a solution prepared with (25) and a part of (26) is added and emulsified. Upon completion of the emulsification, the mixture is cooled to normal temperature to obtain a targeted W/O emulsion emollient cream having a viscosity of 280,000 mPa·s.

### [Example 11] Whitening cream

**[Table 11]**

| | | Blending amount (%) |
|---|---|---|
| (1) | Oleic acid | 3.5 |
| (2) | Isostearic acid (Trade name: Isostearic Acid EX, Kokyu Alcohol Kogyo Co., Ltd.) | 0.5 |
| (3) | Squalane (Trade name: Olive Squalane, Kokyu Alcohol Kogyo Co., Ltd.) | 25.0 |
| (4) | Triethylhexanoin (Trade name: TOG, Kokyu Alcohol Kogyo Co., Ltd.) | 3.0 |
| (5) | Hexyl laurate (Trade name: KAK HL, Kokyu Alcohol Kogyo Co., Ltd. ) | 2.0 |
| (6) | Amide alcohol ester (LHA) of structural formula (I) **(Component A)** | 7.0 |
| (7) | Fragrance | 0.1 |
| (8) | (Vinyl dimethicone/Lauryl dimethicone)crosspolymer/Isododecane mixture (Trade name: KSG-42, Shin-Etsu Chemical Co., Ltd.) | 0.5 |
| (9) | Tranexamic acid | 2.0 |
| (10) | Alkyl-modified carboxyvinyl polymer **(Component B)** (Trade name: Pemulen TR-2, Lubrizol Advanced | 0.15 |
| | Materials, Inc.) | |
| (11) | Methylparaben | 0.1 |
| (12) | Phenoxyethanol | 0.1 |
| (13) | Dimethicone 6cs | 5.0 |
| (14) | Glycerin (Trade name: Triol VE, Kokyu Alcohol Kogyo Co., Ltd. ) | 3.0 |
| (15) | Pentylene glycol (Trade name: Diol PD-V, Kokyu Alcohol Kogyo Co., Ltd. ) | 3.0 |
| (16) | Hypericum perforatum extract | 0.1 |
| (17) | Leontopodium Alpinum extract | 0.1 |
| (18) | Royal jelly extract | 0.1 |
| (19) | Sodium ascorbyl phosphate | 0.1 |
| (20) | Ion exchanged water | Balance |
| (21) | Organically modified clay mineral premix (Trade name: BENTONE ISD V (Isododecane, disteardimonium hectorite, propylene carbonate), Elementis Specialties) | 3.5 |
| (22) | Dextrin myristate (Trade name: Rheopearl MKL, Chiba Flour Milling Co., Ltd. ) | 2.5 |

### <Production method>

(1) to (8), (21) and (22) are uniformly mixed at 80°C (oil phase).

Meanwhile, (9) to (20) are heated to 80°C and mixed uniformly (aqueous phase) . Since tranexamic acid has an action to increase pH, an extremely stable emulsion can be obtained without separately blending an alkali agent.

While gradually adding the aqueous phase to the previously prepared oil phase at 80°C, the mixture is stirred with a disperser.

Upon completion of the emulsification, the mixture is cooled to normal temperature to obtain a targeted W/O emulsion whitening cream having a viscosity of 544,000 mPa·s.

### [Example 12] Whitening beauty essence

**[Table 12]**

| | | Blending amount (%) |
|---|---|---|
| (1) | Di(phytosteryl 2-octyldodecyl)N-lauroyl-L-glutamate (Trade name: Eldew PS-203, Ajinomoto Co., Inc.) | 0.5 |
| (2) | Mineral oil | 5.0 |
| (3) | Squalane (Trade name: Olive squalane, Kokyu Alcohol Kogyo Co., Ltd.) | 15.0 |
| (4) | Isododecane | 10.0 |
| (5) | Isodecyl neopentanoate (Trade name : Neolight 100P, Kokyu Alcohol Kogyo Co., Ltd. ) | 3.0 |
| (6) | Dimethicone 5cs | 1.0 |
| (7) | Amide alcohol ester (LHL) of structural formula (I) **(Component A)** | 5.5 |
| (8) | Alkyl-modified carboxyvinyl polymer **(Component B)** (Trade name: Pemulen TR-1, Lubrizol Advanced Materials, Inc.) | 0.1 |
| (9) | Carboxyvinyl polymer **(Component B)** (Trade name: Carbopol ETD2050 Polymer, Lubrizol Advanced Materials, Inc.) | 0.05 |
| (10) | Sodium hyaluronate | 0.1 |
| (11) | Glycerin (Trade name: Triol VE, Kokyu Alcohol Kogyo Co., Ltd. ) | 5.0 |
| (12) | 1,3-Butylene glycol (Trade name: Haisugarcane BG, Kokyu Alcohol Kogyo Co., Ltd.) | 3.0 |
| (13) | Ethanol | 3.0 |
| (14) | 4-Isobutyl resorcinol | 0.25 |
| (15) | Ascorbic acid glucoside | 1.0 |
| (16) | Fragrance | Suitable amount |
| (17) | Potassium hydroxide | Suitable amount |
| (18) | Sodium pyrosulfite | Suitable amount |
| (19) | Ion exchanged water | Balance |
| (20) | Pentylene glycol (Trade name: Diol PD-V, Kokyu Alcohol Kogyo Co., Ltd. ) | 2.5 |
| (21) | Organically modified clay mineral premix (Trade name: BENTONE ISD V (isododecane, disteardimonium hectorite, propylene carbonate), Elementis Specialties) | 3.5 |
| (22) | Dextrin palmitate (Trade name: Rheopearl KL2, Chiba Flour Milling Co., | 2.5 |
| | Ltd. ) | |
| (23) | Isostearic acid (Trade name: Isostearic acid EX, Kokyu Alcohol Kogyo Co., Ltd.) | 4.0 |

### <Production method>

(1) to (7), (16), (21) to (23) are uniformly dissolved at 80°C (oil phase).

Meanwhile, (8) to (21) are uniformly dissolved at 80°C (aqueous phase). The aqueous phase at 80°C is added to said oil phase at 80°C and stirred with a disperser.

To this, an aqueous solution in which (17) is dissolved in a part of (19) is added and emulsified by stirring with a disperser. Upon completion of the emulsification, a mixed solution of (13) and (10) is added and cooled to normal temperature to obtain a targeted whitening beauty essence having a viscosity of 357,000 mPa·s.

### [Example 13] W/O emulsion sunscreen

**[Table 13]**

| | | Blendin g amount (%) |
|---|---|---|
| (1) | Octyl p-methoxycinnamate | 3.0 |
| (2) | Glyceryl ethylhexanoate di-p-methoxycinnamate | 2.0 |
| (3) | 4-tert-buty-4'-methoxydibenzoylmethane | 2.0 |
| (4) | Tetra(octanoate/p-methoxycinnamate pentaerythritol | 3.0 |
| (5) | Ethylhexyl isononanoate (Trade name: ES108109, Kokyu Alcohol Kogyo Co., Ltd. ) | 5.0 |
| (6) | Dimethicone 20cs | 3.0 |
| (7) | Squalane (Trade name: Olive squalane, Kokyu Alcohol Kogyo Co., Ltd.) | 20.0 |
| (8) | Amide alcohol ester (LHB) of structural formula (I) **(Component A)** | 2.2 |
| (9) | Glycerin (Trade name: Triol VE, Kokyu Alcohol Kogyo Co., Ltd. ) | 0.1 4.0 |
| (10) | Ion exchanged water | Balance |
| (11) | Dipropylene glycol | 1.0 |
| (12) | Methylparaben | 0.2 |
| (13) | Alkyl-modified carboxyvinyl polymer **(Component B)** (Trade name: Pemulen TR-2, Lubrizol Advanced Materials, Inc.) | 0.1 |
| (14) | Fragrance | 0.1 |
| (15) | Triethanolamine | Suitable amount |
| (20) | Pentylene glycol (Trade name: Diol PD-V, Kokyu Alcohol Kogyo Co., Ltd. ) | 2.5 |
| (21) | Organically modified clay mineral premix (Trade name: BENTONE ISD V (isododecane, disteardimonium hectorite, propylene carbonate), Elementis Specialties) | 3.5 |
| (22) | Dextrin palmitate (Trade name: Rheopearl KL2, Chiba Flour Milling Co., Ltd. ) | 2.5 |
| (23) | Isostearic acid (Trade name: Isostearic acid EX, Kokyu Alcohol Kogyo Co., Ltd.) | 4.0 |

### <Production method>

(1) to (8), (14), (21) to (23) are uniformly dissolved at 80°C (oil phase).

Meanwhile, (10) to (20) are uniformly mixed and dissolved at 80°C (aqueous phase).

This aqueous phase is gradually added to said oil phase heated to 80°C, and stirred with a disperser.

Furthermore, (15) is added and emulsified. Upon completion of the emulsification, the mixture is cooled to normal temperature to obtain a targeted W/O emulsion sunscreen having a viscosity of 181,000 mPa·s.

It is also possible to prepare the formulations of Examples 6 to 10, 12 and 13 by pre-adding a neutralizing agent such as potassium hydroxide, triethanolamine, sodium hydroxide, etc. to the aqueous phase.

### [Industrial applicability]

According to the present invention, by combining an amide alcohol ester and a carboxyl group-containing polymer, an agent and a complex can be provided which is capable of preparing emulsions such as O/W emulsion and W/O emulsion using any oil agent.

Such emulsions can be used for applications including cosmetics.

## Claims

1. A method for producing an emulsion composition, wherein an aqueous phase containing a carboxyl group-containing polymer, and an oil phase containing an amide alcohol ester represented by formula (I): 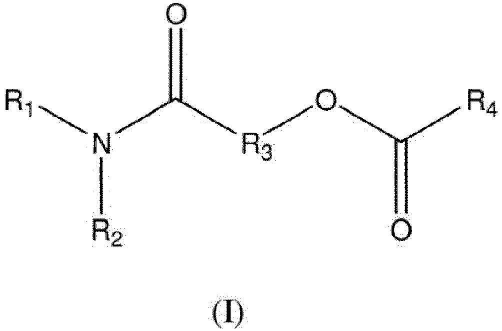 wherein
R₁ is a C6-C22 hydrocarbon group that may be substituted,
R₂ is H, or a C6-C22 hydrocarbon group that may be substituted,
R₃ is a linear or branched C2-C21 hydrocarbon group that may be substituted,
R₄ is a C1-C42 hydrocarbon group that may be substituted; or a group represented by the following formula (II): 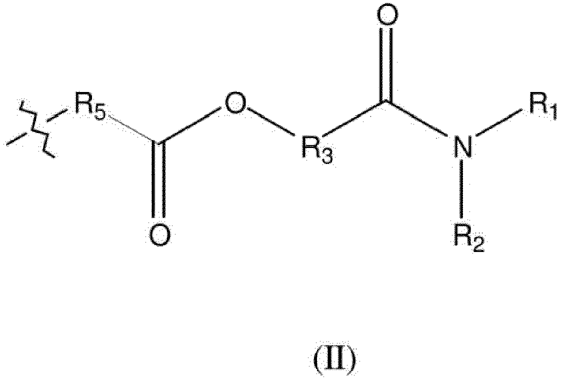 wherein in formula (II),
R₁ to R₃ are the same as the above definitions,
R₅ is a C2-C42 hydrocarbon group that may be substituted; are mixed.

2. The method according to claim 1, which comprises neutralizing by adding a neutralizing agent.

3. The method according to claim 2, wherein the aqueous phase containing the carboxyl group-containing polymer is neutralized by adding a neutralizing agent, and the aqueous phase and the oil phase are mixed.

4. The method according to claim 2, wherein, after mixing the aqueous phase and the oil phase, the mixture is neutralized by adding a neutralizing agent.

5. The method according to any one of claims 1 to 4, wherein the carboxyl group-containing polymer has a molecular weight of 500,000 to 3,000,000 and a carboxyl group content of 50 to 70%.

6. The method according to any one of claims 1 to 5, wherein the carboxyl group-containing polymer is a carboxyvinyl polymer and/or an alkyl-modified carboxyvinyl polymer.

7. The method according to claim 6, wherein the carboxyl group-containing polymer is a carboxyvinyl polymer represented by formula (IV): 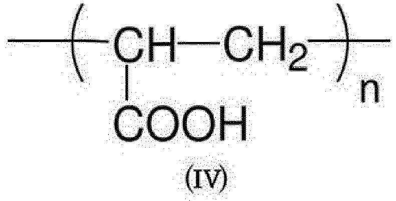 wherein n is an integer,
and/or an alkyl-modified carboxyvinyl polymer represented by formula (V): 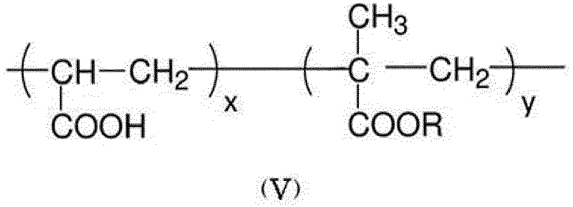 wherein x and y are each independently an integer,
R is a C10-C30 alkyl group.

8. The method according to any one of claims 1 to 7, wherein the amide alcohol ester is an amide alcohol ester of formula (I), wherein
R₁ is a C10-C22 hydrocarbon group,
R₂ is H,
R₃ is a C3-C12 hydrocarbon group,
R₄ is a C1-C22 hydrocarbon group.

9. The method according to claim 8, wherein the amide alcohol is one or more selected from:

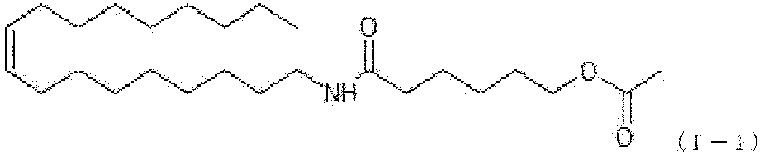

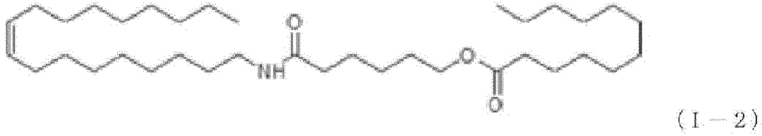

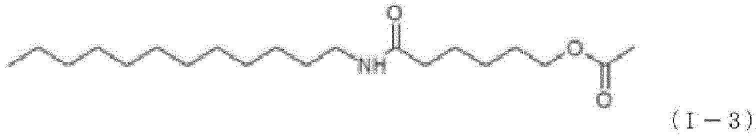

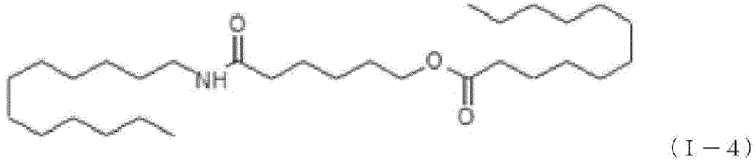

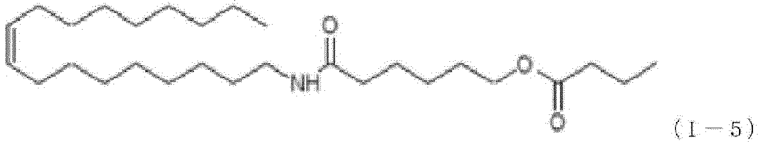

10. An emulsion composition obtained by the method according to any one of claims 1 to 9.

11. An agent containing an amide alcohol ester represented by formula (I): 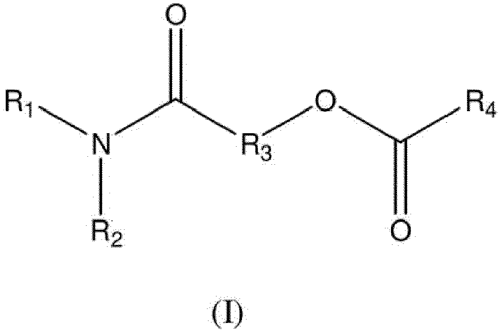 wherein
R₁ is a C6-C22 hydrocarbon group that may be substituted,
R₂ is H, or a C6-C22 hydrocarbon group that may be substituted,
R₃ is a linear or branched C2-C21 hydrocarbon group that may be substituted,
R₄ is a C1-C42 hydrocarbon group that may be substituted; or a group represented by the following formula (II): 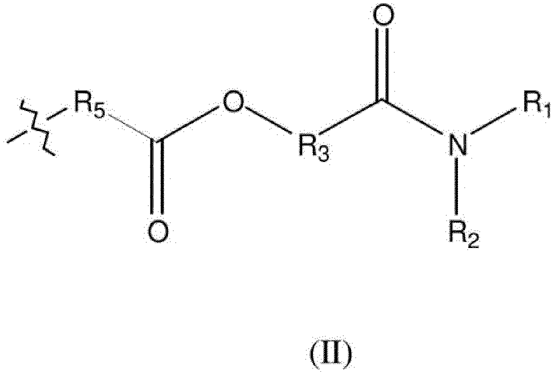 wherein in formula (II),
R₁ to R₃ are the same as the above definitions,
R₅ is a C2-C42 hydrocarbon group that may be substituted; and said agent forms a complex with a carboxyl group-containing polymer and is used for emulsification.

12. An agent containing a carboxyl group-containing polymer, which forms a complex with an amide alcohol ester represented by formula (I): 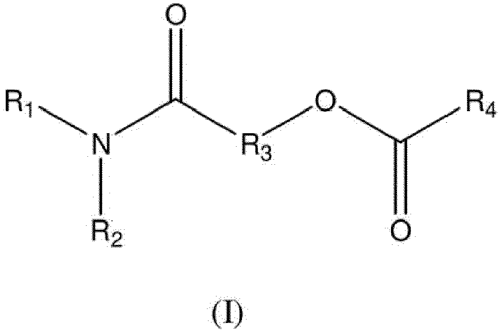 wherein
R₁ is a C6-C22 hydrocarbon group that may be substituted,
R₂ is H, or a C6-C22 hydrocarbon group that may be substituted,
R₃ is a linear or branched C2-C21 hydrocarbon group that may be substituted,
R₄ is a C1-C42 hydrocarbon group that may be substituted; or a group represented by the following formula (II): 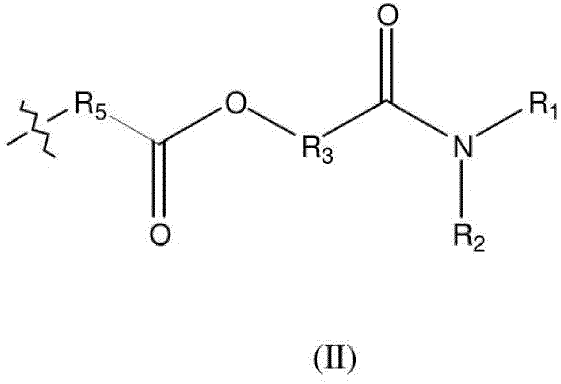 wherein in formula (II),
R₁ to R₃ are the same as the above definitions,
R₅ is a C2-C42 hydrocarbon group that may be substituted; and which is used for emulsification.

13. A complex formed by binding a carboxyl group-containing polymer with an amide alcohol ester represented by formula (I) : 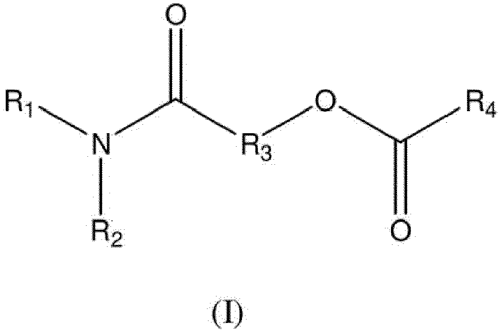 wherein
R₁ is a C6-C22 hydrocarbon group that may be substituted,
R₂ is H, or a C6-C22 hydrocarbon group that may be substituted,
R₃ is a linear or branched C2-C21 hydrocarbon group that may be substituted,
R₄ is a C1-C42 hydrocarbon group that may be substituted; or a group represented by the following formula (II): 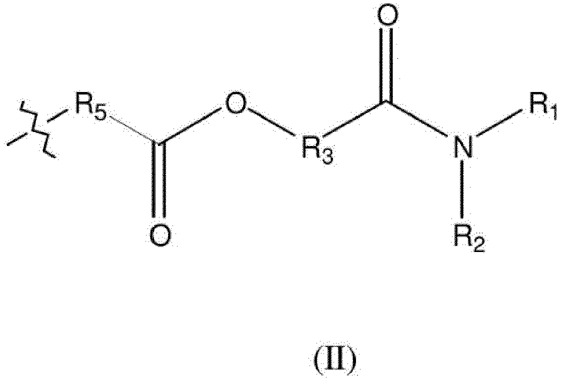 wherein in formula (II),
R₁ to R₃ are the same as the above definitions,
R₅ is a C2-C42 hydrocarbon group that may be substituted.
